# EUROPEAN PATENT SPECIFICATION

(11) **EP 0 294 469 B1**
(45) Date of publication and mention of the grant of the patent: **15.10.1997**
(21) Application number: 88900927.0
(22) Date of filing: 23.12.1987
(51) Int. Cl.: A61K 35/66, A61K 39/00, G01N 33/53, G01N 33/569

(54) **VACCINES AND DIAGNOSTIC ASSAYS FOR HAEMOPHILUS INFLUENZAE**
IMPFSTOFFE UND DIAGNOSETEST FÜR HAEMOPHILUS INFLUENZAE
VACCINS ET ANALYSES DIAGNOSTIQUES POUR L'HAEMOPHILUS INFLUENZAE

(30) Priority: 31.12.1986 US 948364; 02.03.1987 US 20849; 11.12.1987 US 132073
(43) Date of publication of application: 14.12.1988
(60) Divisional application: 96119698.7
(73) Proprietor: AMERICAN CYANAMID COMPANY, Madison, New Jersey 07940 (US)
(72) Inventor: DEICH, Robert, A., Rochester, NY 14625 (US); ZLOTNICK, Gary, Penfield, NY 14526 (US); GREEN, Bruce, Pittsford, NY 14534 (US)
(74) Representative: Warcoin, Jacques
(86) International application number: US8703423
(87) International publication number: WO8804932

(56) References cited:
- EP-A- 0 281 673
- WO-A-86/02360
- US-A- 4 261 968
- US-A- 4 358 535
- US-A- 4 455 296
- US-A- 4 486 539
- BIOLOGICAL ABSTRACTS, vol. 74, no. 4, 1982, page 2556, abstract no. 24608, Philadelphia, PA, US; E.J. HANSEN et al.: "Immunoprotection of rats against Haemophilus influenzae type B disease mediated by monoclonal antibody against a Haemophilus outer membrane protein", & LANCET 1 (8268): 366-368, 1982
- Journal of Clinical Investigation Volume 72, issued 1983 August (New York), R. MUNSON, Jr., et al, "Purification and Comparison of Outer Membrane Protein p2 from Haemophilus Influenzae Type b Isolates", page 677-684, see Abstract, and pages 683-684.
- Infection and Immunity, Volume 41, No. 1, issued 1983 July (Washington, D.C.), J. BURANS, et al., "Induction of Active Immunity with Membrane Fractions from Haemophilus Influenzae Type b", pages 285-293, see Abstract, page 291, Particularly Table 2, and page 293.
- Infection and Immunity, Volume 47 No. 1, issued 1985 January (Washington, D.C.) A. KIMURA, et al., "A Minor High-Molecular-Weight Outer Membrane Protein of Haemophilus Influenzae Type b is a Protective Antigen", pages 253-259, see
- Abstract and page 259.
- Infection and Immunity, Volume 50, No. 1, issued 1985 October (Washington, D.C. ) P. HOLMANS et al., "Cloning and Surface Expression in Escherichia Coli of a Structural Gene Encoding a Surface Protein of Haemophilus Influenzae Type b", pages 236-242, see Abstract, page 236 and page 241.
- Infection and Immunity, Volume 52, No. 3, issued 1986 June (Washington, D.C.), W. THOMAS, et al., "Molecular Cloning of DNA Coding for Outer Membrane Proteins of Haemophilus Influenzae Type b", pages 812-817, see Abstract, pages 812-816.
- Abstracts of the 87th Annual Meeting of the American Society for Microbiology, Volume 87, issued 1987 March (Washington, D.C.), B. NELSON, et al., "Molecular Cloning and Analysis of P6 an Outer Membrane Protein of Haemophilus Influenzae", page 79, Abstract No D-45, see entire Abstract.
- Infection and Immunity, Volume 49 No. 3, issued 1985 September (Washington, D. C.) R. MUNSON, Jr. et al., "Purification and Partial Characterization of Outer Membrane Proteins P5 and P6 from Haemophilus Influenzae Type b", pages 544-549, see Abstract
- Journal of Clinical Investigation Volume 75, issued 1985 May (New York) H. GNEHM et al., "Characterization of Antigens from Nontypable Haemophilus Influenzae Recognized by Human Bactericidal Antibodies", pages 1645-1658 see page 1653 and 1654, Table V.
- Infection and Immunity, Volume 32, No 3, issued 1981 June (Washington, D.C.) E. HANSEN, et al., "Identification of Immunogenic Outer Membrane Proteins of Haemophilus Influenzae Type b in the Infant Rat Model System", pages 1084-1092, see Abstract.
- Journal of Infectious Diseases Volume 152, No. 6, issued 1985 December (Chicago, Illinois), T. MURPHY et al., "Identification of a Specific Epitope of Haemophilus Influenzae on a 16,600 Dalton Outer Membrane Protein", pages 1300- 1307, see Abstract.

## Description

### 1. FIELD OF THE INVENTION

The present invention relates to compositions and methods for the preparation of proteins and peptides associated with the outer membrane of Haemophilus influenzae. More particularly, the invention is directed to compositions and methods for preparation of proteins and peptides related to a class of outer membrane proteins of about 16000 daltons molecular weight of type b and non-typable H. influenzae including PBOMP-1 and PBOMP-2. The proteins and peptides are used as immunogens in vaccine formulations for active immunization and for the generation of antibodies for use in passive immunization and as reagents in diagnostic assays.

The proteins and peptides can be obtained by novel improved methods of purification from H. influenzae or produced using either recombinant DNA or chemical synthetic methods. Additionally, the invention relates to novel DNA sequences and vectors useful for directing expression of PBOMP-1 and PBOMP-2 related proteins and peptides. The nucleotide sequences are used as reagents in nucleic acid hybridization assays.

### 2. BACKGROUND OF THE INVENTION

### 2.1. RECOMBINANT DNA TECHNOLOGY AND GENE EXPRESSION

Recombinant DNA technology involves insertion of specific DNA sequences into a DNA vehicle (vector) to form a recombinant DNA molecule which is capable of replication in a host cell. Generally, the inserted DNA sequence is foreign to the recipient DNA vehicle, i.e., the inserted DNA sequence and the DNA vector are derived from organisms which do not exchange genetic information in nature, or the inserted DNA sequence may be wholly or partially synthetically made. Several general methods have been developed which enable construction of recombinant DNA molecules. For example, U.S. Pat. No. 4,237,224 to Cohen and Boyer describes production of such recombinant plasmids using processes of cleavage with restriction enzymes and joining with DNA ligase by known methods of ligation. These recombinant plasmids are then introduced by means of transformation and replicated in unicellular cultures including procaryotic organisms and eucaryotic cells grown in tissue culture. Because of the general applicability of the techniques described therein, U.S. Pat. No. 4,237,224 is hereby incorporated by reference into the present specification.

Another method for introducing recombinant DNA molecules into unicellular organisms is described by Collins and Hohn in U.S. Pat. No. 4,304,863 which is also incorporated herein by reference. This method utilizes and packaging/transduction system with bacteriophage vectors (cosmids).

Recombinant genes may also be introduced into viruses, such as vaccinia virus. Recombinant viruses can be generated by transfection of plasmids into cells infected with virus.

Regardless of the method used for construction, the recombinant DNA molecule must be compatible with the host cell, i.e., capable of autonomous replication in the host cell or stably integrated into one of the host cell's chromosomes. The recombinant DNA molecule or virus (e.g., a vaccinia virus recombinant) should also have a marker function which allows the selection of the desired recombinant DNA molecule(s) or virus(es). In addition, if all of the proper replication, transcription and translation signals are correctly arranged on the recombinant DNA molecule, the foreign gene will be properly expressed in the transformed bacterial cells, as is the case with bacterial expression plasmids, or in permissive cell lines infected with a recombinant virus or a recombinant plasmid carrying a eucaryotic origin of replication.

Different genetic signals and processing events control many levels of gene expression; for instance, DNA transcription and messenger RNA (mRNA) translation. Transcription of DNA is dependent upon the presence of a promotor which is a DNA sequence that directs the binding of RNA polymerase and thereby promotes mRNA synthesis. The DNA sequences of eucaryotic promotors differ from those of procaryotic promotors. Furthermore, eucaryotic promotors and accompanying genetic signals may not be recognized in or may not function in a procaryotic system and further, procaryotic promotors are not recognized and do not function in eucaryotic cells.

Similarly, translation of mRNA in procaryotes depends upon the presence of the proper procaryotic signals which differ from those of eucaryotes. Efficient translation of mRNA in procaryotes requires a ribosome binding site called the Shine-Dalgarno (SD) sequence on the mRNA. This sequence is a short nucleotide sequence of mRNA that is located before the start codon, usually AUG, which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, 1979, Methods in Enzymology 68:473.

Many other factors complicate the expression of foreign genes in procaryotes even after the proper signals are inserted and appropriately positioned. One such factor is the presence of an active proteolytic system in E. coli and other bacteria. This protein-degrading system appears to selectively destroy "abnormal" or foreign proteins. A tremendous utility, therefore, would be afforded by the development of a means to protect eucaryotic proteins expressed in bacteria from proteolytic degradation. One strategy is to construct hybrid genes in which the foreign sequence is ligated in phase (i.e., in the correct reading frame) with a procaryotic gene. Expression of this hybrid gene results in a fusion protein product (a protein that is a hybrid of procaryotic and foreign amino acid sequences).

Successful expression of a cloned gene requires efficient transcription of DNA, translation of the mRNA and in some instances post-translational modification of the protein. Expression vectors have been used to express genes in a suitable host and to increase protein production. The cloned gene should be placed next to a strong promotor which is controllable so that transcription can be turned on when necessary. Cells can be grown to a high density and then the promotor can be induced to increase the number of transcripts. These, if efficiently translated will result in high yields of protein. This is an especially valuable system if the foreign protein is deleterious to the host cell.

### 2.1.1. E. COLI AS A HOST SYSTEM FOR EXPRESSION

Most plasmid cloning vectors commonly used in E. coli are derivatives of Co1E1-type replicons (for additional information see Oka et al., 1979, Mol. Gen. Genet. 172:151-159). The Co1E1 plasmids are stably maintained in E. coli strains as monomeric molecules with a copy number of about 15-20 copies per chromosome. Various levels of expression of human and animal protein products of foreign genes inserted into these plasmids have been obtained. However, very high expression levels should be obtained in order for the system to become economically feasible to produce foreign protein products.

One way to obtain large amounts of a given gene product is to clone a gene on a plasmid which has a very high copy number within the bacterial cell. In theory, by increasing the number of copies of a particular gene, mRNA levels should also increase which should lead to increased production of the recombinant protein.

### 2.1.2. VACCINIA VIRUS AS AN EXPRESSION VECTOR

Vaccinia virus may be used as a cloning and expression vector. The virus contains a linear double-stranded DNA genome of approximately 187 kb pairs which replicates within the cytoplasm of infected cells. These viruses contain a complete transcriptional enzyme system (including capping, methylating and polyadenylating enzymes) within the virus core which are necessary for virus infectivity. Vaccinia virus transcriptional regulatory sequences (promotors) allow for initiation of transcription by vaccinia RNA polymerase but not by eucaryotic RNA polymerase.

Expression of foreign DNA in recombinant viruses requires the fusion of vaccinia promotors to protein coding sequences of the foreign gene. Plasmid vectors, also called insertion vectors have been constructed to insert the chimeric gene into vaccinia virus. One type of insertion vector is composed of: (1) a vaccinia virus promotor including the transcriptional initiation site; (2) several unique restriction endonuclease cloning sites downstream from the transcriptional start site for insertion of foreign DNA fragments; (3) nonessential vaccinia virus DNA (such as the TK gene) flanking the promotor and cloning sites which direct insertion of the chimeric gene into the homologous nonessential region of the virus genome; and (4) a bacterial origin of replication and antibiotic resistance marker for replication and selection in E. coli. Examples of such vectors are described by MacKett (1984, J. Virol. 49: 857-864)

Recombinant viruses are produced by transfection of recombinant bacterial insertion plasmids containing the foreign gene into cells infected with vaccinia virus. Homologous recombination takes place within the infected cells and results in the insertion of the foreign gene into the viral genome. Recombinant viruses can be screened for and subsequently isolated using immunological techniques, DNA plaque hybridization, or genetic selection. These vaccinia recombinants retain their essential functions and infectivity and can be constructed to accommodate approximately 35 kb of foreign DNA.

Expression of a foreign gene can be detected by enzymatic or immunological assays [e.g., immunoprecipitation, enzyme-linked immunosorbent assay (ELISA), radioimmunoassay, or immunoblotting]. Additionally, naturally occurring membrane glycoproteins produced from recombinant vaccinia infected cells are glycosylated and may be transported to the cell surface. High expression levels can be obtained by using strong promotors or cloning multiple copies of a single gene in appropriate vectors and suitable hosts.

### 2.1.3. BACULOVIRUS AS AN EXPRESSION VECTOR

A baculovirus, such as Autographica californica nuclear polyhedrosis virus (AcNPV) may also be used as a cloning or expression vector. The infectious form of AcNPV is normally found in a viral occlusion. This structure is largely composed of polyhedrin peptide in which virus particles are embedded. Polyhedrin gene expression occurs very late in the infection cycle, after mature virus particles are formed. Therefore polyhedrin gene expression is a dispensable function, i.e., non-occluded virus particles produced in the absence of polyhedrin gene expression are fully active and are capable of infecting cells in culture. According to European Patent Application Serial No. 84105841.5 by Smith et al., a recombinant baculovirus expression vector is prepared by cleaving baculovirus DNA to produce a fragment comprising a polyhedrin gene or portion thereof, inserting this fragment into a cloning vehicle and thereafter inserting the gene to be expressed such that it is under control of the polyhedrin gene promotor. The recombinant transfer vector formed in this way is mixed with baculovirus helper DNA and used to transfect insect cells in culture to effect recombination and incorporation of the selected gene at the polyhedrin gene locus of the baculovirus genome. The resultant recombinant baculovirus is used to infect susceptible insects or cultured insect cells.

### 2.2. HAEMOPHILUS INFLUENZAE AND DISEASE

H. influenzae are divided into two groups. Those strains which possess a known capsule are typed by the serological reaction of the capsule with reference antisera. Types a-f have been identified. Strains which fail to react with any of the reference antisera are known as non-typable.

H. influenzae type b (Hib) is the most frequent cause of neonatal meningitis and other invasive infections in the United States (Fraser et al., 1974, Am. J. Epidemiol. 100:29-34). The major incidence of childhood meningitis occurs between the ages of one and five years. Sixty percent of those meningitis cases due to Hib occur in children under the age of two (Fraser et al., supra).

It is now well established that non-typable H. influenzae (Hi) also cause diseases including pneumonia, bacteremia, meningitis, postpartum sepsis, and acute febrile tracheobronchitis in adults (Murphy et al., 1985, J. Infect. Diseases 152: 1300-1307). Non-typable Hi are a frequent etiologic agent of otitis media in children and young adults, causing about 20 to 40% of all otitis media cases. Children may experience multiple infections due to the same organism since infection confers no long lasting immunity. Current therapy for chronic or repeated occurrences of otitis media includes administration of antibiotics and insertion of tubes to drain the inner ear. Hi strains have also been implicated as primary cause of sinusitis (Cherry J.D. and J.P. Dudley, 1981, in Textbook of Pediatric Infectious Diseases, Feigin and Cherry eds., pp. 103-105). Additionally, non-typable Hi cause neonatal sepsis.

Antiserum produced against the capsular polysaccharide of type b H. influenzae (Hib) which comprises polyribosyl ribitol phosphate (PRP), has been shown to be bactericidal and protective against Hib (Smith et al., 1973, Pediatrics 52:637-644; Anderson, et al. 1972, J. Clin. Inv. 51:31-38). Anti-PRP antibody is ineffective against non-typable H. influenzae infections.

### 2.3. VACCINES AGAINST H. INFLUENZAE

The ideal candidate for a Haemophilus vaccine would have three properties: (a) it would be immunogenic in infants of 2-6 months (b) it would elicit an antibody which would protect against infections caused by typable and non-typable H. influenzae, and (c) it would elicit antibody against a determinant found on the surface of all strains of H. influenzae.

The currently available vaccines which protect against Hib infections consist essentially of PRP, the type b capsular polysaccharide. Purified PRP polysaccharide is immunogenic in children above 18 months of age, but does not elicit a protective antibody response in those younger than 18 months. In general, polysaccharides have been shown to be poor immunogens in children less than about 18 months of age.

To address this problem, various laboratories have begun studies in which PRP is either chemically coupled to a protein carrier molecule (Anderson et al., 1985, Ped. Res. 18:252A) or mixed with protein molecules (Monji et al., 1986, Infect. Immun. 51:865-871) and administered to animals or humans. Conjugation of PRP to protein has been shown to elicit an anti-PRP antibody response in human infants as young as six months, while a mixture of PRP with some proteins has produced anti-PRP antibody in infant animals (Monji et al., supra).

Although the conjugate and admixture vaccine formulations address one difficulty of PRP vaccines, i.e., their inability to protect infants younger than 18 months, they fail to address another major problem of the PRP vaccine. Anti-PRP antibody is ineffective against non-typable H. influenzae, which by definition lack the PRP capsule. Hence there is a long recognized need for a vaccine that will elicit a protective immune response in children of about 18 months and younger against both typable, including type b and non-typable H. influenzae.

One object of the present invention is to provide a vaccine formulation that elicits a protective immune response against typable H. influenzae including type b and non-typable H. influenzae in children under 6 months as well as in older children and adults. The approach of the present invention is to vaccinate with a protein or fragment thereof which is exposed on the surface of Haemophilus. The best candidate is an outer membrane protein (OMP) of H. influenzae. Outer membrane proteins are usually surface exposed molecules. They are composed of protein which is normally immunogenic in infants, and they have been shown to be capable of eliciting protective antibody in other bacterial systems (Sugasawara et al., 1983, Infect. Immun. 42:980-985).

In addition Hi and Hib strains have been shown to have similar OMP profiles (Loeb and Smith, 1980, Infect. Immun. 30:709-717). Antibody to an OMP of Haemophilus could be both bactericidal and opsonic much as anti-PRP has been shown to be bactericidal and opsonic for Hib (Anderson et al., 1972, J. Clin. Invest. 51:31-38; Cates et al., 1985, Infect. Immun. 48:183-189). An outer membrane protein has the additional advantage of being common to Hi and Hib and could protect against both types of bacteria.

### 3. SUMMARY OF THE INVENTION

The present invention is directed to peptides and proteins related to an outer membrane protein of about 16000 daltons molecular weight of Haemophilus influenzae identified by applicants and termed "Praxis Biologics Outer Membrane Protein-1" (PBOMP-1) and to an antigenically related outer membrane protein of about 16000 daltons molecular weight of Haemophilus influenzae also identified by applicants and termed "Praxis Biologics Outer Membrane Protein-2" (PBOMP-2), as well as the molecularly cloned genes or gene fragments which encode these peptides or proteins. The invention is also directed to a substantially pure PBOMP-1 obtained from H. influenzae using novel and improved methods. The peptides or proteins of the present invention may be used as immunogens in vaccine formulations for H. influenzae, or as reagents in diagnostic immunoassays for H. influenzae.

The present invention is also directed to methods for the molecular cloning of genes or gene fragments encoding PBOMP-1 and PBOMP-2 related peptides. These molecularly cloned sequences can then be used in the further construction of other vectors for the encoded peptide products, or in obtaining the PBOMP-1 and PBOMP-2 genes for use in diagnostic assays for H. influenzae based on nucleic acid hybridization.

The peptides or proteins of the present invention may be purified from H. influenzae, or produced using recombinant DNA techniques in any vector-host system, or synthesized by chemical methods. Accordingly, the invention is also directed to the construction of novel DNA sequences and vectors including plasmid DNA, and viral DNA such as human viruses, animal viruses, insect viruses, or bacteriophages which can be used to direct the expression of PBOMP-1 and PBOMP-2 related peptides or proteins in appropriate host cells from which the peptides and proteins may be purified. Chemical methods for the synthesis of PBOMP-1 and PBOMP-2 related peptides and proteins are described.

The PBOMP-1 and PBOMP-2 related peptides and proteins can be used as immunogens in subunit vaccine formulations for use against all pathogenic H. influenzae, including both type b and non-typable H. influenzae. PBOMP-1 and PBOMP-2 related proteins or peptides for subunit vaccine preparations can be obtained by chemical synthesis, purification for H. influenzae or purification from recombinant viruses which produce the PBOMP-1 or PBOMP-2 related peptides or proteins themselves or extracts of cells infected with such recombinant viruses can be used as immunogens in viral vaccine formulations. Since the PBOMP-1 or PBOMP-2 protein will be recognized as "foreign" in the host animal, a humoral and possibly a cell-mediated immune response will be induced, directed against PBOMP-1 or PBOMP-2. In a properly prepared vaccine formulation, this should protect the host against subsequent H. influenzae infections. Moreover, the present subunit vaccine formulations will be compatible with currently available PRP vaccines.

The PBOMP-1 related and/or PBOMP-2 related sequences of the present invention can be used in human medical assays. These include the use of the peptides and proteins of the present invention as reagents in immunoassays such as ELISA tests and radioimmunoassays which are useful as diagnostic tools for the detection of H. influenzae infection in blood samples, body fluid, tissues, etc. The PBOMP-1 encoding and/or PBOMP-2 encoding gene sequences can be used in DNA-DNA or DNA-RNA hybridization assays for similar diagnostic detection of H. influenzae. Additionally, these reagents will provide a valuable tool in elucidating the mechanism of pathogenesis of H. influenzae.

The present invention is directed further to anti-PBOMP-2 monoclonal antibodies which have uses in passive immunization regimes, and in diagnostic immunoassays.

### 4. BRIEF DESCRIPTION OF THE FIGURES

The present invention may be more fully understood by reference to the following detailed description and examples of specific embodiments as well as the appended figures in which:
FIG. 1 represents a restriction map of pGD103, a derivative of pLG339 (see Stoker et al., 1982, Gene 18:335-41).
FIG. 2 (A and B) represents maps of pAA130 which comprises a 5.7 Kb fragment of H. influenzae DNA cloned into pGD1O3. FIG. 2A represents a circular restriction map of pAA130. FIG. 2B represents deletion analysis of the H. influenzae inserted fragment of pAA130. Solid black lines denote remaining H. influenzae DNA in the deletion derivatives. PBOMP phenotype is noted at the right. A gene encoding PBOMP-2 is localized to a 781 bp BstEII-XmnI fragment.
FIG. 3 represents reactivity of whole cell lysates of E. coli JM83 and E. coli JM83 containing pAA130 with polyclonal anti-PBOMP-1 antiserum. Lanes represent: (A) JM83 containing pAA130; (B) JM83 containing pAA130; (C) JM83; (D) JM83; (E) molecular weight standards as displayed in kilodaltons on the right side of the FIG.; and (F) Hi S-2.
FIG. 4 represents the sequencing strategy of the 789 bp BstEII-XmnI fragment of pAA130 showing the origin, direction and extent of sequence determined from each clone. The arrow at the bottom denotes the location of the major open reading frame (ORF).
FIG. 5 represents the nucleotide sequence of the 789 bp BstEII-XmnI fragment of pAA130 which contains the PBOMP-2 gene. The predicted ORF is shown by the underlined sequence. The direction of transcription is denoted by the arrowhead. The two bases designated "N" represent unknown nucleotides.
FIG. 6 represents the deduced amino acid sequence of PBOMP-2. The nucleotide sequence is depicted above and the corresponding amino acid sequence below. The residue enclosed within the box indicates the predicted N-terminal amino acid of the mature form of the protein.
FIG. 7 represents autoradiographic SDS-PAGE analysis of E. coli JM83 cells containing recombinant plasmids pAA130 and pAA152 as well as control E. coli JM83 cells containing pGD103. Lanes represents (1) PAA130; (2) pAA152 and (3) pGD103. The location of a band of about 15,000 daltons molecular weight is noted at the left of the figure.
FIG. 8 (A and B) represents Western blot gel analysis of whole cell lysates of E. coli JM83 containing pAA130 or pAA152 in the presence or absence of globomycin. Molecular weight standards are noted at the left of FIG. 8 (A and B). FIG. 8A represents lysates of cells containing pAA152 which contains the PBOMP-1 gene. Lanes represent: (1) globomycin absent; and (2) globomycin present.
FIG. 8B represents lysates of cells containing pAA130 which contains the PBOMP-2 gene. Lanes represent: (1) globomycin absent; and (2) globomycin present.
FIG. 9 is a schematic representation of the construction of plasmid pPX163 containing the entire coding sequence of PBOMP-2 protein inserted downstream from the lac promoter.
FIG. 10 represents an SDS-PAGE analysis of whole cell lysates of E. coli JM103 containing pPX163 grown in the presence or absence of IPTG. Lanes represent: (1) molecular weight standards: Kilodaltons; (2) lysate of JM103 containing pPXI63 grown without IPTG; and (3) as in Lane 2, grown in the presence of IPTG (5mM) for 4 hours. Arrows show position of three PBOMP-2 reactive bands induced by IPTG.

### 5. DETAILED DESCRIPTION OF THE INVENTION

The present invention is directed to proteins and peptides related to epitopes of an approximately 16000 dalton molecular weight outer membrane protein of H. influenzae, i.e., PBOMP-1 and of a related approximately 16000 dalton molecular weight outer membrane protein of H. influenzae, i.e., PBOMP-2. The apparent molecular weights as determined using SDS-PAGE reflect the total molecular weights of the mature (i.e., proteolytically processed) forms, including any post-translational modification(s) (e.g., fatty acylation, acetylation, etc.). The proteins and peptides of the invention can be produced using recombinant DNA methods or by chemical synthesis. Additionally, the proteins and peptides of the invention can be obtained in substantially pure form from cultures of H. influenzae using novel and improved methods of isolation and purification. The PBOMP-2 proteins and peptides specifying epitopes of H. influenzae can be used as immunogens in various vaccine formulations to protect against infection with H. influenzae, an etiological agent of bacterial meningitis, otitis media, epiglottitis, pneumonia, etc. The vaccine formulations are effective against both H. influenzae typable strains including types a, b, c, d, e, and f as well as non-typable H. influenzae strains.

The present invention further relates to the nucleotide sequence(s) of the genes encoding the PBOMP-2 proteins as well as the amino acid sequences of the PBOMP-2 proteins and polypeptide fragments thereof.

According to one embodiment of the present invention, recombinant DNA techniques are used to insert nucleotide sequences encoding PBOMP-2 epitopes into expression vectors that will direct the expression of these sequences in appropriate host cells. These expression vector host cell systems can be used to produce PBOMP-2 and related proteins and peptides. The gene products can be purified from cells in culture and used as immunogens in subunit vaccine formulations. Alternatively, the amino acid sequence of PBOMP-2 proteins and peptides may be deduced from the H. influenzae nucleotide sequences contained in recombinants that express immunogenic PBOMP-2 related proteins and peptides. These proteins and peptides may then be chemically synthesized and used in synthetic subunit vaccine formulations.

Where the expression vector that expresses the PBOMP-2 sequence is a recombinant virus, the virus itself may be used as a vaccine. Infectious recombinant viruses that express the PBOMP-2 proteins and peptides and that do not cause disease in a host can be used in live virus vaccine preparations to provide substantial immunity. Alternatively, inactivated virus vaccines can be prepared using "killed" recombinant viruses that express the PBOMP-2 proteins and peptides.

The present invention is further directed to polyvalent antiserum and monoclonal antibody against PBOMP-2 as well as methods for use of such immunoglobulin for passive immunization, and diagnostic assays for H. influenzae.

For the purpose of description, the method of the invention can be divided into the following stages:
(1) isolation and purification of PBOMP protein; (2) partial amino acid sequencing of PBOMP; (3) molecular cloning of genes or gene fragments encoding PBOMP-2, including insertion of the genes or gene fragments into expression vectors and identification and purification of the recombinant gene products; (4) nucleotide sequencing of the gene encoding PBOMP-2; and (5) determination of the immunopotency of the PBOMP-2 proteins and related products through production of antibodies against purified and recombinant protein and peptide products. The method further encompasses (6) formulation of vaccines and (7) diagnostic assays for detection of PBOMP-2 gene or gene product (and hence H. influenzae) in samples of body fluids.

### 5.1. MOLECULAR CLONING OF GENES OR GENE FRAGMENTS ENCODING PBOMP-2

### 5.1.1. ISOLATION OF GENES ENCODING PBOMPs

A 16000 dalton molecular weight (MW) OMP has been detected, both by sodium dodecyl sulfate polyacrylamide gel electrophoresis (SDS-PAGE) and Western Blot analysis in all H. influenzae strains tested (currently several hundred). Monoclonal antibody data indicate that this protein is highly conserved (Murphy et al., 1986, Infect. Immun. 54:774-49). Thus, any H. influenzae strain could serve as the source for the PBOMP genes. Since many H. influenzae strains contain no detectable plasmids or inducible prophages, the PBOMP genes are probably chromosomal. Accordingly, the first step in the molecular cloning of DNA sequences encoding PBOMPs is the isolation of such sequences from H. influenzae chromosomal DNA. Hereinafter, DNA encoding H. influenzae genes will be referred to as "Hi DNA", and DNA encoding PBOMPs sequences will be referred to as "PBOMP DNA".

In order to generate Hi DNA fragments, the Hi DNA may be cleaved at specific sites using various restriction enzymes. Alternatively, one may use low concentrations of DNAase I to fragment the DNA, or the DNA can be physically sheared, as for example, by sonication. The linear DNA fragments may then be separated according to size by standard techniques, including, but not limited to: agarose and polyacrylamide gel electrophoresis, column chromatography (e.g., molecular sieve or ion exchange chromatography) or velocity sedimentation in sucrose gradients.

Any restriction enzyme or combination of restriction enzymes may be used to generate the Hi DNA fragment(s) containing the PBOMP sequences provided the enzyme(s) does not destroy the immunopotency of the PBOMP gene products. For example, the antigenic site of a protein can consist of from about 7 to about 14 amino acids. Thus, a protein of the size of the PBOMP peptides may have many discrete antigenic sites and therefore, many partial PBOMP polypeptide gene sequences could code for an antigenic site. Consequently many restriction enzyme combinations may be used to generate DNA fragments, which, when inserted into an appropriate vector are capable of directing the production of PBOMP specific amino acid sequences comprising different antigenic determinants.

Once the DNA fragments are generated, identification of the specific DNA fragment containing the PBOMP gene may be accomplished in a number of ways.

The DNA sequences containing the PBOMP genes may be identified by hybridization of the Hi DNA fragments with a synthetic oligonucleotide probe. Redundant synthetic oligonucleotide probes are constructed based upon the amino acid sequence of peptide fragments of the PBOMP protein. These synthetic probes can be radio-labeled with ³²P-adenosine triphosphate and used to screen Hi DNA libraries for clones containing PBOMP-specific gene sequences (see Anderson et al., 1983, Proc. Nat'l Acad. Sci. USA 80:6838-42).

Alternatively, the PBOMP gene DNA may be identified and isolated after insertion into a cloning vector in a "shotgun" approach. A large number of vector-host systems known in the art may be used. Vector systems may be either plasmids or modified viruses. Suitable cloning vectors include, but are not limited to the viral vectors such as lambda vector system gtll, gt WES.tB, Charon 4, and plasmid vectors such as pBR322, pBR325, pACYC177, pACYC184, pUC8, pUC9, pUC18, pUC19, pLG339, pR290, pKC37, pKC101 and other similar systems. The vector system must be compatible with the host cell used. Recombinant molecules can be introduced into cells via transformation, transfection or infection.

When Hi DNA containing a PBOMP gene or gene fragment is inserted into a cloning vector and used to transform appropriate host cells many copies of the PBOMP gene or gene fragment can be generated. This can be accomplished by ligating the Hi DNA fragment into a cloning vector which has complementary cohesive termini. If, however, the complementary restriction sites are not present, the ends of the DNA molecules may be modified. Such modification includes producing blunt ends by digesting back single-stranded DNA termini or by filling the single-stranded termini so that the ends can be blunt-end ligated. Alternatively, any site desired may be produced by ligating nucleotide sequences (linkers) onto the DNA termini. These ligated linkers may comprise specific chemically synthesized oligonucleotides encoding restriction site recognition sequences. For example, according to the DNA modification procedure of Maniatis, (see Maniatis et al., 1982, Molecular Cloning, Cold Spring Harbor Laboratory, pp. 107-114) sheared DNA is treated with a restriction methylase (for example, M. EcoRI) and ligated to synthetic DNA linkers which encode a restriction site for that enzyme. The DNA is then treated with restriction endonuclease to cleave the terminal linkers (but not the modified internal restriction sites) and ligated to the appropriate vector arms. In an alternative method, the cleaved vector and PBOMP DNA fragment may be modified by homopolymeric tailing.

Identification of a cloned PBOMP gene can be accomplished by establishing a chromosomal gene bank of Hi in a vector system and screening individual clones for the production of PBOMP-2 or PBOMP-2 related protein by any of the methods described herein, including, but not limited to specific reaction with polyclonal or monoclonal antibodies against PBOMPS.

### 5.1.2. INSERTION OF PBOMP GENES INTO EXPRESSION VECTORS

The nucleotide sequences coding for PBOMPs or portions thereof, are inserted into an appropriate expression vector, i.e., a vector which contains the necessary elements for the transcription and translation of the inserted protein-coding sequences. A variety of host-vector systems may be utilized to express the protein-encoding sequence(s). Primarily the vector system must be compatible with the host cell used. Host-vector systems include but are not limited to the following: bacteria transformed with bacteriophage DNA, plasmid DNA or cosmid DNA; microorganisms such as yeast containing yeast vectors; mammalian cell systems infected with virus (e.g., vaccinia virus, adenovirus, etc.); insect cell systems infected with virus (e.g., baculovirus). The expression elements of these vectors vary in their strength and specificities. Depending upon the host-vector system utilized, any one of a number of suitable transcription and translation elements can be used.

In order to obtain efficient expression of the gene (or a portion of the gene), a promotor must be present in the expression vector. RNA polymerase normally binds to the promotor and initiates transcription of a gene or a group of linked genes and regulatory elements (called an operon). Promoters vary in their "strength", i.e., their ability to promote transcription. For the purpose of expressing a cloned gene, it is desirable to use strong promotors in order to obtain a high level of transcription and, hence, expression of the gene. Depending upon the host cell system utilized, any one of a number of suitable promotors may be used. For instance, when cloning in E. coli, its bacteriophages or plasmids, promotors such as the lac promotor, trp promotor, recA promotor, ribosomal RNA promotor, the P_{R} and P_{L} promotors of coliphage lambda and others including but not limited to lacUV5, ompF, bla, lpp and the like, may be used to direct high levels of transcription of adjacent DNA segments. Additionally, a hybrid trp-lacUV5 (tac) promotor or other E. coli promotors produced by recombinant DNA or other synthetic DNA techniques may be used to provide for transcription of the inserted gene.

Bacterial host cell strains and expression vectors may be chosen which inhibit the action of the promotor unless specifically induced. In certain operons the addition of specific inducers is necessary for efficient transcription of the inserted DNA; for example, the lac operon is induced by the addition of lactose or IPTG (isopropylthio-beta-D-galactoside). A variety of other operons, such as trp, pro, etc., are under different controls. The trp operon is induced when tryptophan is absent in the growth media; and the P_{L} promotor of lambda can be induced by an increase in temperature in host cells containing a temperature sensitive lambda repressor, e.g., cI857. In this way, greater than 95% of the promotor-directed transcription may be inhibited in uninduced cells. Thus, expression of the genetically engineered PBOMP protein or peptide thereof may be controlled. This is important if the protein product of the cloned gene is lethal or detrimental to host cells. In such cases, transformants may be cultured under conditions such that the promotor is not induced, and when the cells reach a suitable density in the growth medium, the promotor can be induced for production of the protein.

One such promotor/operator system is the so called "tac" or trp-lac promotor/operator system (Russell and Bennett, 1982, Gene 20:231-243; DeBoer, European Patent Application, 67,540 filed May 18, 1982). This hybrid promotor is constructed by combining the -35 b.p. (-35 region) of the trp promoter and the -10 b.p. (-10 region or Pribnow box) of the lac promotor (the sequences of DNA which are the RNA polymerase binding site). In addition to maintaining the strong promoter characteristics of the tryptophan promoter, tac is also controlled by the lac repressor.

When cloning in a eucaryotic host cell, enhancer sequences (e.g., the 72 bp tandem repeat of SV40 DNA or the retroviral long terminal repeats or LTRs, etc.) may be inserted to increase transcriptional efficiency. Enhancer sequences are a set of eucaryotic DNA elements that appear to increase transcriptional efficiency in a manner relatively independent of their position and orientation with respect to a nearby gene. Unlike the classic promotor elements (e.g., the polymerase binding site and the Goldberg-Hogness "TATA" box) which must be located immediately 5' to the gene, enhancer sequences have a remarkable ability to function upstream from, within, or downstream from eucaryotic genes; therefore, the position of the enhancer sequence with respect to the inserted gene is less critical.

Specific initiation signals are also required for efficient gene transcription and translation in procaryotic cells. These transcription and translation initiation signals may vary in "strength" as measured by the quantity of gene specific messenger RNA and protein synthesized, respectively. The DNA expression vector, which contains a promoter, may also contain any combination of various "strong" transcription and/or translation initiation signals. For instance, efficient translation in E. coli requires a Shine-Dalgarno (SD) sequence about 7-9 bases 5' to the initiation codon (ATG) to provide a ribosome binding site. Thus, any SD-ATG combination that can be utilized by host cell ribosomes may be employed. Such combinations include but are not limited to the SD-ATG combination from the cro gene or the N gene of the coliphage lambda, or from the E. coli tryptophan E, D, C, B or A genes. Additionally, any SD-ATG combination produced by recombinant DNA or other techniques involving incorporation of synthetic nucleotides may be used.

Any of the methods previously described for the insertion of DNA fragments into a vector may be used to ligate a promotor and other control elements into specific sites within the vector.

Accordingly, H. influenzae genetic sequences containing those regions coding for the PBOMP proteins or peptides can be ligated into an expression vector at a specific site in relation to the vector promotor and control elements so that when the recombinant DNA molecule is introduced into a host cell the foreign genetic sequence can be expressed (i.e., transcribed and translated) by the host cell. The recombinant DNA molecule may be introduced into appropriate host cells (including but not limited to bacteria, virus, yeast, mammalian cells or the like) by transformation, transduction or transfection (depending upon the vector/host cell system). Transformants are selected based upon the expression of one or more appropriate gene markers normally present in the vector, such as ampicillin resistance or tetracycline resistance in pBR322, or thymidine kinase activity in eucaryotic host systems. Expression of such marker genes should indicate that the recombinant DNA molecule is intact and is replicating. Expression vectors may be derived from cloning vectors, which usually contain a marker function. Such cloning vectors may include, but are not limited to the following: SV40 and adenovirus, vaccinia virus vectors, insect viruses such as baculoviruses, yeast vectors, bacteriophage vectors such as lambda gt-WES-lambda B, Charon 28, Charon 4A, lambda gt-1-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1, RP4, PBR328 and the like.

Transfer of drug resistance factors between H. influenzae and E. coli via conjugation (Stuy, 1979, J. Bact. 139:520-529); and transformation (Mann, 1979, Plasmid 2:503-505) and cloning of Haemophilus chromosomal genes in E. coli (Mann et al., 1980, Gene 3:97-112) indicate that at least some genes can be efficiently expressed in both organisms; and that the basic mechanisms of transcriptional and translational control may be similar.

In the particular embodiment in the examples of the present invention, an E. coli plasmid system was chosen as the expression vector. The invention, however, is not limited to the use of such E. coli expression vector.

Genetic engineering techniques could also be used to further characterize and/or adapt the cloned gene. For example, site directed mutagenesis of the gene encoding a PBOMP protein could be used to identify regions of the protein responsible for generation of protective antibody responses. It could also be used to modify the protein in regions outside the protective domains, for example, to increase the solubility of the protein to allow easier purification.

### 5.1.3. IDENTIFICATION AND PURIFICATION OF THE EXPRESSED GENE PRODUCTS

Expression vectors containing foreign gene inserts can be identified by three general approaches: (1) DNA-DNA hybridization using probes comprising sequences that are homologous to the foreign inserted gene; (2) presence or absence of "marker" gene functions (e.g., resistance to antibiotics, transformation phenotype, thymidine kinase activity, etc.); and (3) expression of inserted sequences based on the physical, immunological or functional properties of the gene product.

Once a recombinant which expresses a PBOMP gene is identified, the gene product should be analyzed. Immunological analysis is especially important because the ultimate goal is to use the gene products or recombinant viruses that express such products in vaccine formulations and/or as antigens in diagnostic immunoassays.

A variety of antisera are available for analyzing immunoreactivity of the product, including, but not limited to polyvalent antisera and monoclonal antibodies.

Identification of the proteins and peptides of the invention requires that the PBOMP related protein or peptide be immunoreactive to a variety of antibodies directed against PBOMP or its analogs and derivatives.

The protein or peptide should be immunoreactive whether it results from the expression an entire PBOMP gene sequence, a portion of the gene sequence or from two or more gene sequences which are ligated to direct the production of fusion proteins. This reactivity may be demonstrated by standard immunological techniques, such as radioimmunoprecipitation, radioimmune competition, ELISA or immunoblots.

Once the H. influenzae PBOMP related protein is identified, it may be isolated and purified by standard methods including chromatography (e.g., ion exchange, affinity, and sizing column chromatography), centrifugation, differential solubility, or by any other standard techniques for the purification of proteins.

Alternatively, once an immunoreactive H. influenzae PBOMP related protein produced by a recombinant is identified, the amino acid sequence of the immunoreactive protein can be deduced from the nucleotide sequence of the chimeric gene contained in the recombinant. As a result, the protein can be synthesized by standard chemical methods known in the art (e.g., see Hunkapiller et al., 1984, Nature 310:105-111).

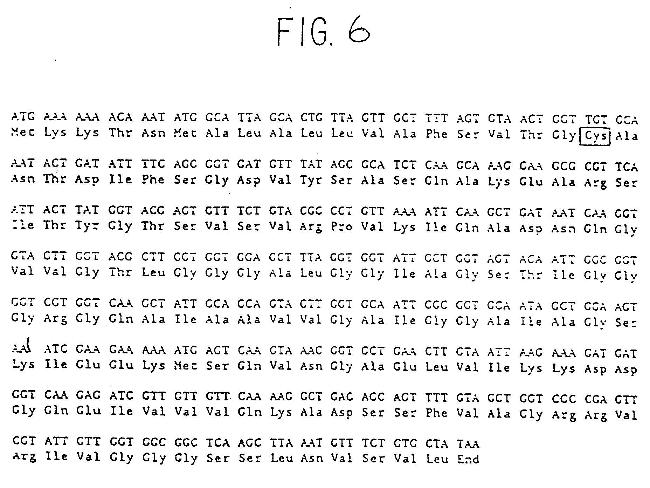

In a particular embodiment of the present invention such peptides, whether produced by recombinant DNA techniques or by chemical synthetic methods, include but are not limited to all or part of the amino acid sequences substantially as depicted in FIG. 6 including altered sequences in which functionally equivalent amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the non-polar (hydrophobic) amino acids include glycine, alanine, leucine, isoleucine, valine, proline, phenylalanine, tryptophan and methionine. The polar neutral amino acids include serine, threonine, cysteine, tyrosine, asparagine, and glutamine. The positively charged (basic) amino acids include arginine, lysine and histidine. The negatively charged (acidic) amino acids include aspartic and glutamic acid.

### 5.2. NUCLEOTIDE SEQUENCING OF PBOMP GENES

Once the fragments of DNA containing the PBOMP genes have been identified, the actual nucleotide sequences of these genes can be ascertained by sequence analysis of the DNA. The sequential order of the base pairs can be determined by either of two methods, the method of Maxam and Gilbert (Maxam and Gilbert, 1980, Methods in Enzymology, 65:49) or the dideoxy method (Sanger et al., 1977, Proc. Nat'l Acad. Sci. USA 74:5463). The actual start and stop signals of the PBOMP genes can be ascertained by analysis of the nucleotide sequence for open reading frames (Rosenberg et al., 1979, Ann. Rev. Genet. 13:319). If more than one open reading frame is found on a particular DNA fragment, the identity of the actual gene could be confirmed by comparing the predicted amino acid sequence of the gene product to the amino acid sequence of the PBOMP. The location of the proper reading frame may also be determined by the use of gene fusions.

### 5.3. DETERMINATION OF IMMUNOPOTENCY OF PBOMPs

Experience with antibodies to the capsular polysaccharide of type b Haemophilus influenzae i.e., PRP, shows that the ability of the antibodies to kill the bacteria in in vitro assays and to protect against challenge with Hib in animal model systems is closely correlated with the ability to elicit a protective immune response in human infants.

Anti-PBOMP antibodies elicited in response to the PBOMP proteins and peptides of this invention can be tested using similar in vitro assay systems and animal model system to demonstrate the ability to kill both Hi and Hib cells and to protect in animal model systems from challenge with Hib. The results from these systems should show a similar correlation with the potential of each of the PBOMPs to elicit a protective immune response and to serve in a vaccine for human infants, children and adults.

An in vitro complement mediated bactericidal assay system (Musher et al., 1983, Infect. Immun. 39:297-304; Anderson et al., 1972, J. Clin. Invest. 51:31-38) which has been used previously for measuring bactericidal activity of antibodies of PRP and lipopolysaccharide (LPS) against H. influenzae could be used to determine whether or not antibody directed against a particular PBOMP peptide or fragment thereof has bactericidal activity against type b H. influenzae and non-typable H. influenzae. These assays can be performed against a relatively large number of clinical isolates of both types of bacteria to determine whether a broad range of strains are killed.

Further data on the ability of a PBOMP to elicit a protective antibody response may be generated by use of the infant rat meningitis model system (Smith et al., 1973, Infect. Immun. 8:278-290). Infant rats challenged before the sixth day of life, with a suitable dose of H. influenzae type b develop bacteremia and a fatal meningitis similar to that seen in human infants. If antibody which is bactericidal against a challenge strain is used to passively immunize infant rats prior to challenge, then they are protected from meningitis and death. Antibodies directed against the current vaccine type b Haemophilus, PRP, are protective in the infant rat model system. Passive protection against type b Haemophilus meningitis could be demonstrated by immunizing infant rats with rabbit polyclonal anti-PBOMP antibody and subsequently challenging the rats with a lethal dose of H. influenzae type b.

Data on the ability of antibody to a particular PBOMP to protect against Hi could be obtained using the chinchilla otitis media animal model system. (Barenkamp et al., 1986, Infect. Immun. 52:572-78). In this animal model, chinchillas are challenged by inoculation of the inner ear canal with Hi. An otitis media much like that seen in humans develops. Chinchillas, which have been immunized, either actively with Hi OMP's, or passively with antibody directed against Hi OMP's are protected against aural challenge with Hi. (Barenkamp et al., supra). This animal model system could be used to demonstrate the ability of antibody to a PBOMP to protect against Hi.

It is possible to demonstrate that anti-PBOMP antibodies are capable of additive protection along with anti-PRP antibodies by use of the infant rat animal model. Anti-PBOMP antibodies diluted to a point at which they no longer are capable of protecting infant rats against challenge with Hib, mixed with a similar dilution of anti-PRP antibodies, may show additive protection and thus prevent death of infant rats. This additive protection might be useful for a potential combination vaccine composed of PRP, or a fragment or conjugate thereof, and the PBOMP or a fragment thereof.

### 5.4. FORMULATION OF A VACCINE

Many methods may be used to introduce the vaccine formulations described below into a human or animal. These include, but are not limited to: intradermal, intramuscular, intraperitoneal, intravenous, subcutaneous and intranasal routes of administration.

### 5.4.1. SUBUNIT VACCINE FORMULATIONS

One purpose of the present invention is to provide proteins or polypeptide fragments related to outer membrane proteins of H. influenzae, PBOMPs including PBOMP-2 and related proteins and peptides, which are used as immunogens in a subunit vaccine to protect against meningitis and other disease symptoms of H. influenza infections. Subunit vaccines comprise solely the relevant immunogenic material necessary to immunize a host. Vaccines made from genetically engineered immunogens, chemically synthesized immunogens and/or immunogens comprising authentic substantially pure H. influenza PBOMPs isolated as described herein, which are capable of eliciting a protective immune response are particularly advantageous because there is no risk of infection of the recipients. Thus, the PBOMP related protein or fragment thereof may be purified from recombinants that express the PBOMP epitopes. Such recombinants include any of the previously described bacterial transformants, yeast transformants, or cultured cells infected with recombinant viruses that express the PBOMP epitopes (see Sections 5.1 and 5.2, supra). Alternatively, the PBOMP related protein or peptide may be chemically synthesized. To this end, the amino acid sequence of such a protein or peptide can be deduced from the nucleotide sequence of the gene which directs its expression (see Section 5.2, supra).

Whether the immunogen is purified from recombinants or chemically synthesized, the final product is adjusted to an appropriate concentration and formulated with any suitable vaccine adjuvant. Suitable adjuvants include, but are not limited to: surface active substances, e.g., hexadecylamine, octadecylamine, octadecyl amino acid esters, lysolecithin, dimethyl-dioctadecylammonium bromide, N, N-dioctadecyl-N'-N-bis (2-hydroxyethyl-propane diamine), methoxyhexadecylglycerol, and pluronic polyols; plyamines, e.g., pyran, dextransulfate, poly IC, polyacrylic acid, carbopol; peptides, e.g., muramyl dipeptide, dimethylglycine, tuftsin; oil emulsions; and mineral gels, e.g., aluminum hydroxide, aluminum phosphate, etc. The immunogen may also be incorporated into liposomes, or conjugated to polysaccharides and/or other polymers for use in a vaccine formulation.

In yet another embodiment of this mode of the invention, the PBOMP related protein or peptide is a hapten, i.e., a molecule that is antigenic in that it reacts specifically or selectively with cognate antibodies, but is not immunogenic in that it cannot elicit an immune response. In such case, the hapten may be covalently bound to a carrier or immunogenic molecule; for example, a large protein such as protein serum albumin will confer immunogenicity to the hapten coupled to it. The hapten-carrier may be formulated for use as a subunit vaccine.

### 5.4.2. VIRAL VACCINE FORMULATIONS

Another purpose of the present invention is to provide either a live recombinant viral vaccine or an inactivated recombinant viral vaccine which is used to protect against meningitis and other disease symptoms of H. influenzae. To this end, recombinant viruses are prepared that express PBOMP related epitopes (see Sections 5.1 and 5.2, supra). Where the recombinant virus is infectious to the host to be immunized but does not cause disease, a live vaccine is preferred because multiplication in the host leads to a prolonged stimulus, therefore, conferring substantially long-lasting immunity. The infectious recombinant virus when introduced into a host can express the PBOMP related protein or polypeptide fragment from its chimeric gene and thereby elicit an immune response against H. influenzae antigens. In cases where such an immune response is protective against subsequent H. influenzae infection, the live recombinant virus itself may be used in a preventative vaccine against H. influenzae infection. Production of such recombinant virus may involve both in vitro (e.g., tissue culture cells) and in vivo (e.g., natural host animal) systems. For instance, conventional methods for preparation and formulation of smallpox vaccine may be adapted for the formulation of live recombinant virus vaccine expressing a PBOMP related protein or polypeptide.

Multivalent live virus vaccines can be prepared from a single or a few infectious recombinant viruses that express epitopes of organisms that cause disease in addition to the epitopes of H. influenzae PBOMPs. For example, a vaccinia virus can be engineered to contain coding sequences for other epitopes in addition to those of H. influenzae PBOMPs. Such a recombinant virus itself can be used as the immunogen in a multivalent vaccine. Alternately, a mixture of vaccinia or other viruses, each expressing a different gene encoding for different epitopes of PBOMPs and/or other epitopes of other disease causing organisms can be formulated in a multivalent vaccine.

Whether or not the recombinant virus is infectious to the host to be immunized, an inactivated virus vaccine formulation may be prepared. Inactivated vaccines are "dead" in the sense that their infectivity has been destroyed, usually by chemical treatment (e.g., formaldehyde). Ideally, the infectivity of the virus is destroyed without affecting the proteins which carry the immunogenicity of the virus. In order to prepare inactivated vaccines, large quantities of the recombinant virus expressing the PBOMP related protein or polypeptide must be grown in culture to provide the necessary quantity of relevant antigens. A mixture of inactivated viruses which express different epitopes may be used for the formulation of "multivalent" vaccines. In certain instances, these "multivalent" inactivated vaccines may be preferable to live vaccine formulation because of potential difficulties with mutual interference of live viruses administered together. In either case, the inactivated recombinant virus or mixture of viruses should be formulated in a suitable adjuvant in order to enhance the immunological response to the antigens. Suitable adjuvants include, but are not limited to: surface active substances, e.g., hexadecylamine, octadecyl amino acid esters, octadecylamine, lysolecithin, dimethyl-dioctadecylammonium bromide, N, N-dicoctadecyl-N'-N-bis (2-hydroxyethyl-propane diamine), methoxyhexadecylglycerol, and pluronic polyols; plyamines, e.g., pyran, dextransulfate, poly IC, polyacrylic acid, carbopol; peptides, e.g., muramyl dipeptide, dimethylglycine, tuftsin; oil emulsions; and mineral gels, e.g., aluminum hydroxide, aluminum phosphate, etc.

### 5.4.3. PASSIVE IMMUNITY AND ANTI-IDIOTYPIC ANTIBODIES

Instead of actively immunizing with viral or subunit vaccines, it is possible to confer short-term protection to a host by the administration of pre-formed antibody against an epitope of H. influenzae. Thus, the vaccine formulations can be used to produce antibodies for use in passive immunotherapy. Human immunoglobulin is preferred in human medicine because a heterologous immunoglobulin may provoke an immune response to its foreign immunogenic components. Such passive immunization could be used on an emergency basis for immediate protection of unimmunized individuals exposed to special risks, e.g., young children exposed to contact with bacterial meningitis patients. Alternatively, these antibodies can be used in the production of anti-idiotypic antibody, which in turn can be used as an antigen to stimulate an immune response against H. influenza PBOMP epitopes.

### 5.5. DIAGNOSTIC ASSAYS

Yet another purpose of the present invention is to provide reagents for use in diagnostic assays for the detection of PBOMP antigens (and hence H. influenzae) in various body fluids of individuals suspected of H. influenzae infection.

### 5.5.1. IMMUNOASSAYS

In one mode of this embodiment, the PBOMP related proteins and peptides of the present invention may be used as antigens in immunoassays for the detection of H. influenzae in various patient tissues and body fluids including, but not limited to: blood, spinal fluid, sputum, etc.

The antigens of the present invention may be used in any immunoassay system known in the art including, but not limited to: radioimmunoassays, ELISA assays, "sandwich" assays, precipitin reactions, gel diffusion precipitin reactions, immunodiffusion assays, agglutination assays, fluorescent immunoassays, protein A immunoassays and immunoelectrophoresis assays, to name but a few.

### 5.5.2. NUCLEIC ACID HYBRIDIZATION ASSAY

In another mode of this embodiment, the novel nucleotide sequence of the genes encoding the PBOMP related protein and peptides of the present invention may be used as probes in nucleic acid hybridization assays for the detection of H. influenzae in various patient body fluids, including but not limited to: blood, spinal fluid, sputum, etc.

The nucleotide sequences of the present invention may be used in any nucleic acid hybridization assay system known in the art including, but not limited to: Southern blots (Southern, 1975, J. Mol. Biol. 98:508); Northern blots (Thomas et al., 1980, Proc. Nat'l Acad. Sci. USA 77:5201-05); colony blots (Grunstein et al., 1975, Proc. Nat'l Acad. Sci. USA 72:3961-65), etc. The following series of Examples are presented for purposes of illustration and not by way of limitation on the scope of the present invention.

### 6. EXAMPLES: ISOLATION AND CHARACTERIZATION OF NATURAL AND RECOMBINANT DNA-DERIVED PBOMPs

### 6.1. GENERAL PROCEDURES USED FOR PREPARATION OF RECOMBINANT PLASMIDS

### 6.1.1. CONDITIONS FOR RESTRICTION ENZYME DIGESTIONS

Restriction endonucleases were purchased from BRL (Bethesda, MD), IBI (New Haven, CT), New England Biolabs (Beverly, MA), or US Biochemical Corporation (Cleveland, OH).

Restriction enzyme digestions were carried out by suspending DNA in the appropriate restriction buffer, adding restriction endonuclease, and incubating for an appropriate period of time to ensure complete digestion. One unit of enzyme is defined as the amount required to completely digest 1.0 ug of phase lambda DNA in 1 hour in a total reaction mixture of 20 ul volume. Buffers used with the various enzymes are listed below:

Low salt buffer used for ClaI, HpaI, HpaII, and KpnI digestions consisted of: 10 mM Tris (pH 8.0), 10 mM MgCl₂ and 10 mM dithiothreitol (DTT).

Medium salt buffer used for AluI, AvaI, EcoRII, EcoRV, HaeII, HaeIII, HincIII, HindIII, PstI, Sau3AI, SphI, SstI, SstII, TaqI, and XhoI digestions consisted of: 50 mM Tris (pH 8.0), 10 mM MgCl₂, 50 mM NaCl, and 10 mM DTT.

High salt buffer used for BamHI, EcoRI, PvuI, SalI and XbaI digestions consisted of: 50 mM Tris (pH 8.0), 10 mM MgCl₂, 150 mM NaCl and 10 mM DTT.

The buffer used for SmaI digestions consisted of: 10 mM Tris (pH 8.0), 20 mM KCL, 10 mM MgCl₂, and 10 mM DTT. All restriction digestions were carried out at 37°C except TaqI, which was carried out at 60°C.

### 6.1.2. GEL PURIFICATION OF DNA FRAGMENTS

After restriction enzyme digestions, DNA fragments of varying sizes were separated and purified using gel electrophoresis in low melting temperature agarose (FMC LGT agarose) using 50 mM Tris-acetate 1 mM EDTA buffer pH 7.8 at 10 volts/cm. Agarose concentrations varied from 0.8% to 1.5% depending on the size of fragments to be recovered. DNA bands were visualized by ethidium bromide fluorescence and cut out of the gel. DNA was recovered by melting the agarose at 65°C, adding 4 volumes of 0.65 M NaCl, 10 M Tris (pH 8.0), 1 mM EDTA to bring the mixture to a final concentration of 0.5 M NaCl, loading the DNA onto a NACS column (BRL, Bethesda, MD) equilibrated with 0.5 mM NaCl, 10 mM Tris pH 8.0, 1 mM EDTA (loading buffer), washing the column with 3-5 volumes of loading buffer, and eluting with 2-3 volumes 2 M NaCl, 10 mM Tris pH 8.0, 1 mM EDTA. The DNA eluate was diluted 1:1 with double distilled H₂0 and precipitated with 3 volumes of ethanol. The pellet was washed with 70% ethanol, vacuum dried, and resuspended in 10 mM Tris-HCl buffer, pH 7.5 containing 1 mM EDTA (TE buffer).

### 6.1.3. DNA LIGATION

All ligations were accomplished using T4 DNA ligase. T4 DNA ligase was purchased from BRL (Bethesda, MD), United States Biochemicals (Cleveland, OH) or Boehringer (Indianapolis, IN). One unit (U) of T4 DNA ligase is defined as the amount required to yield 50% ligation of HindIII fragments of bacteriophage lambda DNA in 30 minutes at 16°C in 20 ul volume ligase buffer at a 5'-DNA termini concentration of 0.12 uM (300 ug/ml). DNA ligations were performed in ligase buffer consisting of: 50 mM Tris (pH 7.5), 10 mM MgCl₂, 10 mM DTT, 1 mM adenosine triphosphate). Normally a DNA concentration ranging from 20-30 ug/ml, and a molar ratio of vector to insert of 1:1 was used. T4 DNA ligase was added at a ratio of 1 U per 20 ul reaction volume. Incubations were carried out for 18-24 hours. Temperatures used were 15°C for cohesive end ligations, and 22°C for blunt end ligations. If sufficient material was available, ligations were checked by analyzing a portion of the reaction mixture by agarose gel electrophoresis.

### 6.1.4. PROTEIN IMMUNOBLOT ANALYSIS (WESTERN BLOT)

Proteins were fixed to nitrocellulose sheets for immunoblot analysis by various techniques, depending on the particular application. Phage plaques were transferred from agar plates by gently placing a sterile 8.1 cm diameter nitrocellulose disc onto the surface of a 10 cm diameter phage titer plate. The sheet was allowed to wet completely, positions were marked by punching through the filter with a sterile needle, and the filter was lifted after two minutes.

Colony blots were performed by transferring bacterial colonies to a nitrocellulose sheet, allowing the colonies to grow by placing the sheet (colony side up) on nutrient agar for 4 to 6 hours, and exposing the sheet to chloroform vapor for 30 minutes to lyse the colonies. Protein gel transfers were performed by placing an SDS-PAGE gel containing the protein mixture to be analyzed on a nitrocellulose sheet and applying horizontal electrophoresis in a Hoeffer Transphor apparatus at 0.5 A for 14 hours in 25 mM Tris 0.38 M glycine pH 8.8 buffer.

Once protein transfer was complete, filters were soaked in 50 mM Tris (pH 8.0), 150 mM NaCl, 5% nonfat dry milk (BLOTTO) at 37°C for one hour in all cases, except colony blots. When colony blots were performed, the filters were soaked overnight at 4°C in BLOTTO containing 1 mg/ml lysozyme to digest cell debris. Filters were then absorbed with a first antibody probe at an appropriate dilution (determined by trial and error) in BLOTTO for 3 hours at 37°C, washed three times for 15 minutes with BLOTTO, absorbed with horseradish peroxidase conjugated second antibody (Kirkegaard and Perry, Gaithersburg, MD) at a dilution of 1:500 in BLOTTO for one hour at 37°C and washed with BLOTTO three times for 15 minutes. Filters were placed in 50 mM Tris (pH 7.0), 150 mM NaCl, .01% hydrogen peroxide; and 0.06% 4-Chloro-1-naphthol (Sigma Chemical Co., St. Louis, MO) in methanol was added. If no blue color developed within 20 minutes, the reaction was considered negative. The reaction was stemmed by transferring the filter to distilled water and blotting dry.

### 6.1.5. GENE FUSIONS

Fusions of a gene or gene fragment encoding a PBOMP protein or peptide thereof to another gene such as the gene encoding alkaline phosphatase (PhoA) were carried out as described by Manoil and Beckwith (1985, Proc. Nat'l Acad. Sci. USA 82:8129-8133). Recombinant plasmids were introduced into an E. coli strain containing a deletion of the native PhoA gene and carrying a derivative of transposon Tn5 (TnPhoA) which contains an alkaline phosphatase gene, which lacks both a promotor and a membrane transport signal sequence, inserted into the left terminal repeat of Tn5 on an F-prime plasmid. Hence, production of active alkaline phosphatase enzyme requires transposition of TnPhoA such that the PhoA gene is fused in frame into an actively transcribed gene containing a membrane transport signal peptide. Such transpositions were detected by plating cells in the presence of 40 ug/ml 5-Bromo-4-Chloro-3-Indolyl Phosphate (XP, Sigma Chemical Co., St. Louis, MO). In the presence of this dye, colonies producing active alkaline phosphatase enzyme appear intensely blue in color while colonies which lack active alkaline phosphatase appear white.

### 6.1.6. DNA FILTER HYBRIDIZATION ANALYSIS (SOUTHERN BLOT)

DNA filter hybridization analysis was carried out according to the procedure of Southern (1975, J. Mol Biol. 98: 508). DNA to be analyzed by filter hybridization was digested with appropriate restriction endonuclease(s) and separated by agarose gel electrophoresis in 0.8% Agarose (SeaKem Portland, ME) using 90 mm Tris-borate, 8 mM EDTA buffer and 10 volts/cm. DNA in the gel was denatured by soaking the gel in 1.5 M NaCl/0.5 M NaOH for 1 hour and neutralized by soaking in 1.5 M NaCl/1.0 M Tris-HCl for 1 hour. Denatured DNA was transferred to nitrocellulose filter paper by blotting. Following transfer of DNA, filter were washed with 6 X SSC (prepared by dilution from a 20X SSC stock containing 175.5 g NaCl and 88.2 g Na citrate/liter) and air dried. DNA fragments were fixed to the filter by baking at 80°C for 2 hours under vacuum.

DNA hybridization probes were prepared by nick translation according to the procedure of Rigby et al., (1977, J. Mol. Biol., 113:237-244). DNA for the probe (1-2 ug) was dissolved in 100 u nick-translation buffer (50 mm Tris-HCl, pH 7.4, 10 mm MgSO₄ 10 mM DTT, 5 ug/ml bovine serum albumin, and 20 um each dGTP, dCTP, and dTTP). To this reaction mixture, 100 uCi of alpha ³²P-dATP (Amersham, 2-3000 Ci/mmole), 1.0 ng of deoxyribonuclease I (Sigma Chemical Co., St. Louis, MO) and 10 U E. coli DNA polymerase I (Boehringer) were added and the mixture incubated at 15°C for 45 minutes. The reaction was stopped by the addition of EDTA to 50 MM and heating to 65°C for 10 minutes to inactivate the enzymes. The labeled DNA was precipitated by addition of three volumes of ethanol and resuspended to 50 ul of 0.3 M ammonium acetate (NH₄OAc). The sample was loaded onto a 1 ml Biogel P-50 spin column equilibrated with 0.3 M NH₄OAc and eluted by centrifugation at 500 x g for 5 minutes. The column was washed with 100 ul 0.3 M NH₄OAc and the eluates combined and precipitated with three volumes of ethanol. The labelled DNA pellet was vacuum dried, resuspended in TE buffer, radioactive incorporation measured in a Beckman (LS9000) scintillation counter by Cherenkov scattering.

For hybridization, filters with bound DNA were wetted with 6 x SSC and prehybridized with 6 X SSC/ 0.5% SDS/5X Denhardt's solution/100 ug/ml tRNA at 68°C for 2 hours to block excess binding capacity of the filter (1 X Denhardt's solution is .02% Ficoll., 0.02% polyvinylpyrrolidone, .02% bovine serum albumin in water). The hybridization reaction was carried out in the same buffer to which 0.01 M EDTA and 5-10,000,000 CPM (Cherenkov) labelled probe was added. The probe solution was heated to 90°C for 10 minutes prior to application to denature the DNA strands, cooled to 68°C, and incubated with the filter at 68°C for 18-24 hours. After hybridization, filters were washed with several changes of 0.1 X SSC/0.5% SDS at 68°C in order to remove nonspecifically bound probe. Under the conditions used, DNA homologies of greater than or equal to 90% would show positive binding of the DNA probe. Filters were air dried and exposed on Kodak XAR film at -70°C using Dupont CRONEX 'Lightning Plus' intensifying screens.

### 6.2. CLONING THE PBOMP GENES OF H. INFLUENZAE

The source of H. influenzae chromosomal DNA for cloning of the PBOMP genes was either H. influenzae KW20b (HiKW2Ob), a derivative of a non-encapsulated Rd stain of Hi transformed to type b+ by DNA from strain b-Eagan (Moxon et al, 1984, Clin. Invest. 73:298-306) or H. influenzae S2, (Hi S2), a spontaneous capsule-minus mutant of Hib Eagan.

To generate a phage lambda library, chromosomal DNA from Hi was sheared to an average length of about 15000 base pairs (bp), blunt ended by treatment with T4 DNA polymerase, modified with EcoRI DNA methylase, ligated to synthetic EcoRI linkers, and cloned into the recombinant Lambda phage vector Charon 4.

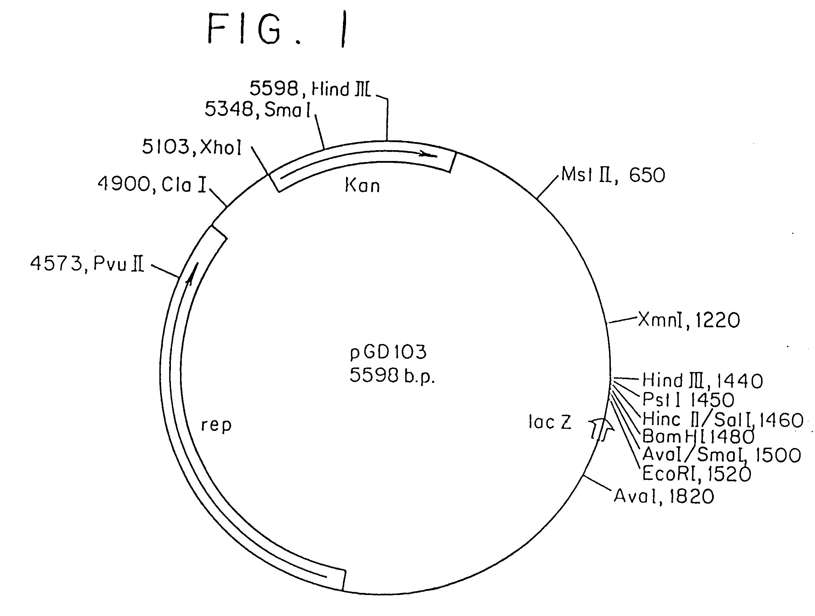

To generate a plasmid library chromosomal DNA of Hi S2 was partially cleaved with Sau3A, the 3-8 kilobase (kb) length restriction fragments thus generated were isolated, and ligated into plasmid vector pGD103 at the BamHI restriction site. This plasmid is a derivative of pLG339 (See FIG. 1; see also Stoker et al., 1982, Gene 18:335-41) and is carried in 6-8 copies/cell. It also contains the lac Z-alpha peptide and polylinker region from plasmid pUC8; and therefore, if transformed into an appropriate E. coli strain (such as JM83), allows selection of recombinant plasmids by screening for loss of the Lac+ phenotype. If cloned in the proper orientation, it is also possible that a cloned gene which is poorly expressed in E. coli could come under control of the strong, regulated lac promotor. E. coli containing recombinant plasmids were screened for production of PBOMPs using a pooled mixture of monoclonal antibodies or polyclonal anti-PBOMP antiserum.

### 6.2.1. CONSTRUCTION OF HI PLASMID LIBRARY

It is possible that the PBOMP protein is not expressed on or is incompatible with lambda phage. In order to test this we constructed a plasmid chromosomal library of Hi 52. Cloning of E. coli OMP genes on high copy number plasmids has been shown to be toxic (see, for example, Beck et al., 1980, Nucleic Acid Res. 8:3011-3024). In order to avoid this problem, we used the low copy number plasmid pGD103 (see FIG. 1).

Chromosomal DNA from a Hi S2 was partially digested with restriction endonuclease Sau3A (BRL, Bethesda, MD). Five hundred micrograms of DNA was digested in 5 ml restriction buffer with 50 units of Sau3A for 1 hour at 37°C. Fragments were separated by velocity sedimentation in a Beckman SW28 rotor on 10-40% sucrose gradients containing 10 mM Tris (pH 8.0), 1 mm EDTA, 1 M NaCl at 140,000 x g for 24 hours. Two ml fractions were collected and aliquots analyzed by agarose gel electrophoresis. Fractions containing restriction fragments of 3-8 Kb in length were pooled and concentrated in TE buffer. Plasmid pGD103 DNA was digested with BamHI endonuclease and treated with calf alkaline phosphatase (Boehringer, Indianapolis, IN) (1 Unit/ug DNA, 37°C x 30 minutes in restriction buffer). DNA was purified from the reaction mixture by phenol extraction and ethanol precipitation and resuspended in TE buffer. Since BamHI and Sau3A restriction enzymes form cohesive ends, no further treatment of DNAs prior to ligation was necessary.

About twenty-five ug each of Hi S2-Sau3A digested DNA and pGD103/BamHI/CAP digested DNA were mixed in 500 ml ligation buffer containing 25 U T4-ligase (Boehringer, Indianapolis, IN) and incubated at 15°C for 18 hours. A 20 ul aliquot of the reaction mixture was analyzed by agarose-gel electrophoresis in order to verify the ligation reaction (starting material was run in an adjacent lane) The ligation mixture was then transformed into competent E. coli JM83 (se Maniatis et al., 1982, Molecular Cloning, Cold Spring Harbor Laboratory, p. 250) incubated for 1 hour in LB-broth at 37°C, and plated on LB-agar plates containing 50 ug/ml kanamycin sulfate (Sigma Chemical Co., St. Louis, MO) and 40 ug/ml 5-Bromo-4-chloro-3-indolyl-Beta-D-galactopyranoside (X-gal, BRL Bethesda, MD), and incubated 24 hours at 37°C. About 50% of the kanamycin resistant (kanR) colonies which developed were white (Lac-), indicating insertion of S2 DNA into the BamHI site in the lac region of pGD103 (Lac+ non-recombinants are blue). Ten white colonies were selected at random, amplified, and shown to contain plasmids 4-8 Kb larger than pGD103 with insertions at the vector BamHI site.

One thousand five hundred and twenty-five white colonies were picked, amplified individually, and stored frozen at -70°C in LB broth containing 18% sterile glycerol in 96-well microtiter dishes.

### 6.2.2. CONSTRUCTION OF HIB LAMBDA GENE BANK

High molecular weight chromosomal DNA from Hi KW20b was suspended in TE buffer at a concentration of 200 ug/ml and sheared to an average length of 15000 bp by passage through a 25 gauge needle. Protruding ends were removed by treatment with T4 DNA polymerase in 50 mM Tris, (pH 8.8), 10 mM MgCl₂, 20 mM (NH₄)₂SO₄ 10 mM DTT, 50 uM dATP, dCTP, dGTP, and dTTP) at 37°C for 20 minutes. DNA was then modified with EcoRI DNA methylase (1 U/ug DNA) (BRL Bethesda, MD), in 100 mM Tris (pH 8.0), 10 mM EDTA, 0.1 mM S-adenosyl-methionine) for 3 hours at 37°C. Methylation of DNA was verified by removing 1 ug of DNA from the reaction, mixing with 1 ug of unmodified lambda DNA and digesting in 20 ul of high salt restriction buffer with 5 units of EcoRI for 1 hour at 37°C. Under these conditions, the modified Hi DNA was not digested, while the added lambda DNA was digested to completion.

Twenty micrograms of modified Hi DNA was ligated to 1 ug chemically synthesized EcoRI linkers (BRL Bethesda, MD) in a 100 ul reaction mixture using T4 DNA ligase (5U). After 18 hours, the reaction was stopped by heating to 60°C for 20 minutes, NaCl was added to a final concentration of 150 mM, and the mixture was digested with 10 U EcoRI for 6 hours. Modified Hi DNA plus linker was separated from cleaved and unligated linkers by agarose gel electrophoresis as described above.

Prepared Hi DNA was mixed with the left and right EcoRI fragments of lambda Charon 4 DNA at a 1:1:1 molar ratio and ligated with T4 DNA polymerase for 18 hours. The ligated DNA mixture was packaged into Lambda phage particles using an in vitro packaging reaction. Five ug of ligated DNA in 4 ul H₂0 was added to 7 ul of 20 mM Tris (pH 8), 10 mM 2-mercaptoethanol (2-ME), 3 mM MgCl₂, 1 ul of 10 mM Tris, pH 7.5, 1 mM Spermidine, 2 mM putrescine, 10 mM MgCl₂ 1.5 mM ATP, 5 mM 2ME and 5 ul of sonic extract from E. coli BHB2690 (-1 imm 434, cI^{ts}, b2.red3, Eam 15, Sam7) lysate (Hohn et al., 1977, Proc. Nat'l Acad. Sci. 74:3259). The reaction mixture was incubated at 22°C for 1 hour and packaged phages were separated by centrifugation in a 3 M to 5 M CsCl [in 10 mm Tris (pH 7.5), 10 mM MgCl_{2,} .02% gelatin (TMG buffer)] step gradient for 250,000 x g for 4 hours in a Beckman SW50.1 rotor. Phage were removed from the interface and dialyzed against TMG. Tittering of the phage thus prepared indicated a library of 25-30,000 independent clones of the Hi genome had been generated. The phage library was amplified by plate amplification using E. coli KH802 as a phage cost to yield 5 ml of phage suspension containing 10⁻⁹ plaque forming units (PFU)/ml.

### 6.3. ISOLATION OF PBOMP GENES

### 6.3.1. ISOLATION OF A PBOMP GENE ENCODING A PROTEIN WHICH REACTS WITH POLYCLONAL ANTI-PBOMP-1 ANTISERA

The amplified phage library prepared as described in section 6.2.1. was diluted to 1-2000 PFU in one ml TMG and 50 ul of E. coli KH802 (5 x 10⁹ cells/ml) were added. The mixture was incubated at 37°C for 20 minutes and plated with 3 ml soft agar on agar plates containing NZYCM medium: 10 g NZ Amine A, 5.0 g NaCl, 2.0 g MgSO4.7H₂0, 5 g Yeast Extract, 1 g casamino acids (per liter). Plates were incubated overnight, chilled to 4°C for 30-60 minutes, and plaques were transferred to nitrocellulose. Filters were probed with polyclonal anti-PBOMP-1 as described above in Section 6.1.4. Several positive plaques were detected in this manner. However, no positive plaques were detected when PBOMP-1 monoclonal antibodies were used as a probe. Positive plaques were picked from the plate and amplified by growth in E. coli KH802. Clones were verified by SDS-PAGE gel/Western Blot analysis of phage lysates. All positive clones expressed a protein of apparent molecular weight 16000 daltons which reacted with polyclonal antibody to PBOMP-1. This protein was not present in control lysates of Charon 4 phage. In similar experiments, lysates from the positive clones failed to react with monoclonal antibodies to PBOMP-1.

One positive phage, designated lambda 16-3 was selected for further analysis. This phage isolate was amplified by growth in E. coli KH802 in NZYCM broth, recovered by precipitation with 20% Polyethylene glycol 6000 and banded in cesium chloride equilibrium gradients (4 M CsCl in TMG, Beckman SW50.1 rotor, 300,000 x g for 24 hours). Phage DNA was isolated by treatment with 0.1% SDS and 20 ug/ml proteinase K (Sigma Chemical Co., St. Louis, MO) at 55°C for 1 hour followed by extraction with phenol and ethanol precipitation.

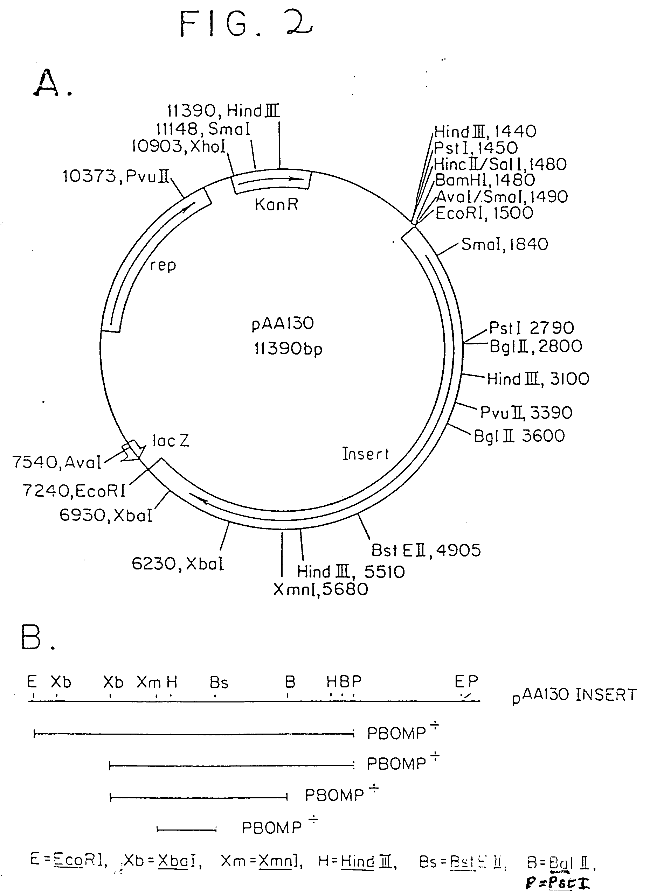

The lambda 16-3 DNA was digested with EcoRI and a partial physical map of the Hi chromosomal insert was obtained. EcoRI fragments of the insert were isolated and subcloned into plasmid vector pGD103. Clones carrying fragments expressing the PBOMP-1 cross-reactive 16000 dalton protein were identified by Western blot transfer analysis of cell lysates. One of these was designated pAA130. FIG. 2 represents a restriction map of this plasmid having an 5.7 Kb fragment from Hi DNA cloned into pGD103 plasmid.

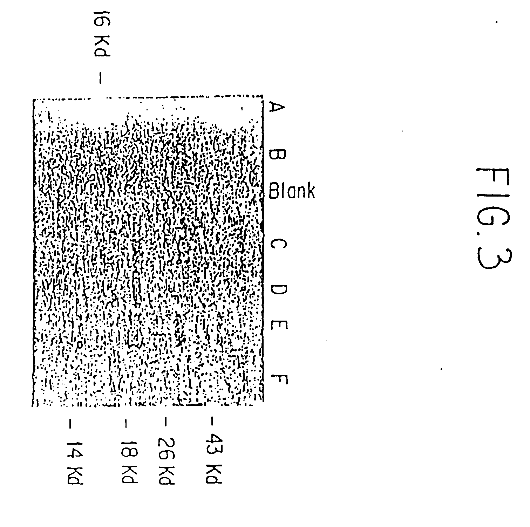

Monoclonal antibodies against PBOMP-1 did not react with the 16000 dalton protein expressed from pAA130 (data not shown). The protein expressed by this recombinant plasmid was recognized, however, by polyclonal anti-PBOMP-1 antisera (see FIG. 3 for example).

Analysis of minicells (Achtman et al., supra) carrying pAA130 indicated that the cloned Hib DNA codes for proteins of apparent molecular weights of 16000 and 17000 daltons. The labelled 16000 dalton protein was specifically immunoprecipitated by polyclonal anti-PBOMP-1 (data not shown). Thus, plasmid pAA130 directs the expression of a 16000 dalton molecular weight PBOMP.

An internal deletion generated by excision of DNA inserted between the unique PstI site of the insert and the single PstI in the polylinker did not affect the expression of the cross-reacting protein. The XbaI fragment of this plasmid was deleted by a similar method and expression of the PBOMP cross-reacting protein was retained (FIG.2). An additional deletion derivative of this plasmid was generated by religation of the two internal BglIII sites and this derivative also retained expression of the PBOMP cross-reactive protein.

The 781 base pair BstEII-XmnI fragment was cloned by isolating the fragment from a low melting point agarose gel, filling in the BstEII end with Klenow fragment of DNA Polymerase I and cloning the fragment into the HincII site of pGD103. Western blot analysis using polyclonal anti-PBOMP-1 showed that this plasmid retained expression of the 16000 dalton PBOMP. As with pAA130, the PBOMP produced from this plasmid failed to react with monoclonal antibodies to PBOMP-1.

As an independent method of verifying the location of this PBOMP gene, the large EcoRI-PvuII fragment of pAA130 was ligated with the EcoRI-PvuII fragment of pLG339 to generate a new tetracycline resistant plasmid designated pAA136. This plasmid expressed the PBOMP as verified by Western blots. This plasmid was transformed into an E. coli strain with deletion of the chromosomal alkaline phosphatase gene (phoA) and carrying the transposible element TnPhoA. Three independent transpositions of the TnPhoA element into pAA136 which restored alkaline phosphatase activity were isolated. The sites of the TnPhoA insertions into pAA136 were determined using the unique DraI restriction site near the left terminal region of TnPhoA and the HindIII, BstEII, XmnI, and PstI sites of pAA136. All three insertions were determined to fall within the BstEII-XmnI fragment of pAA136. All three TnPhoA insertions were in the same orientation indicating that transcription of the PBOMP gene is directed from the BstEII site towards the XmnI site in pAA136. All three TnPhoA transpositions resulted in loss of the 16000 protein detected by polyclonal anti-PBOMP-1 antiserum as detected by Western blots. One fusion generated a new band on Western blots at 60000 dalton which was detected by polyclonal anti-PBOMP-1 antiserum. This size is within the predicted range of fusion proteins that might be generated by fusion of alkaline phosphatase (45000 daltons MW) to a 16000 dalton MW protein. Restoration of PhoA activity in these transpositions verifies that the PBOMP protein contains a peptide signal for membrane transport; and hence, is probably a membrane protein.

The TnPho fusions were secluded by subcloning the junction between TnPhoA and the Hi cloned DNA sequences into M13. In all cases the PhoA coding sequences were determined to be in frame with the predicted open reading frame for the PBOMP-2 gene of pAA130 (see Section 6.4.1 infra).

### 6.4. DETERMINATION OF THE NUCLEOTIDE SEQUENCE OF PBOMP GENES

### 6.4.1. SEQUENCING STRATEGY FOR THE PBOMP GENE OF pAA130

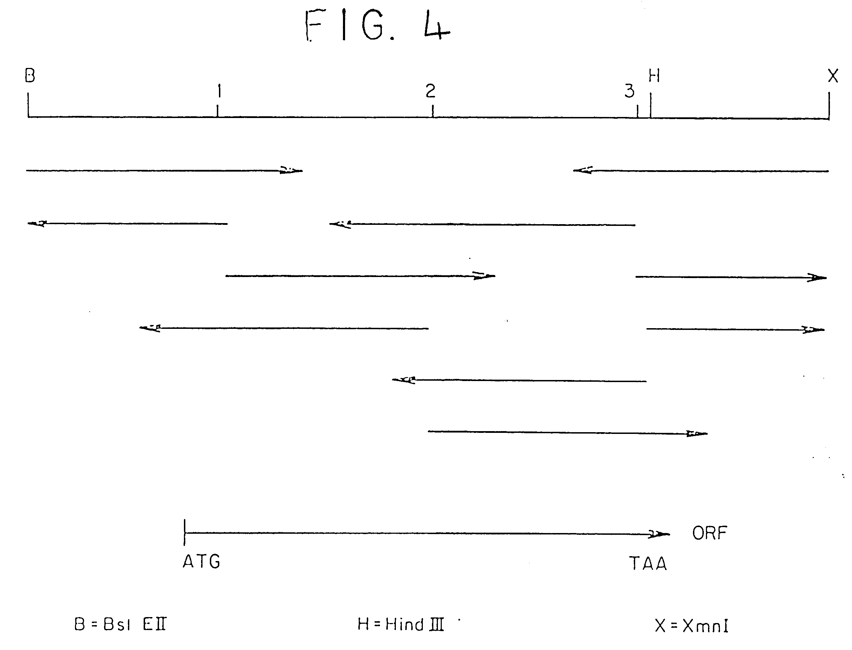

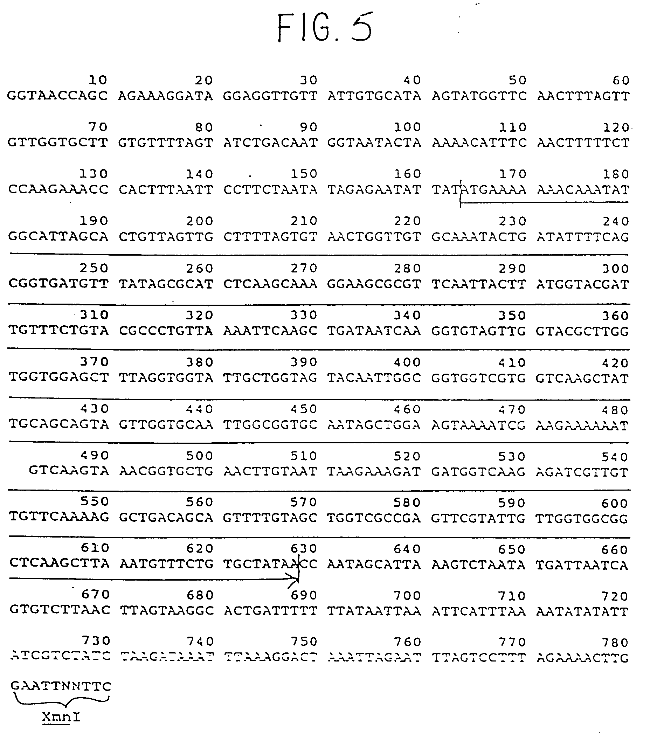

The nucleotide sequence of the PBOMP gene of pAA130 was determined by dideoxynucleotide sequencing (Sanger, et al. supra) of the 789 base pair BstEII-Xmnl fragment of pAA130 after subcloning into M13 mpl8 and mpl9 phage. These recombinant phage are designated M18001 and M19001 respectively. The universal 17 base oligonucleotide sequencing primer (New England Biolabs) was used to determine the sequence from both ends of the BstEII-XmnI fragment (see FIG. 4). Two additional oligonucleotides were synthesized and used as primers for dideoxynucleotide sequencing (M18PRI, M19PR2). All other sequencing primers were made at Praxis Biologics, Rochester, N.Y. on an Applied Biosystems 380 B DNA synthesizer. The primers were made on a 1 umole controlled pore glass column with beta-cyanoethyl phosphate protecting group chemistry. The yield of oligonucleotide was sufficiently pure to allow the use of the primers directly from the column without further purification. The two synthetic oligonucleotide primers bind to sequences approximately 200 nucleotides in from each end of the fragment as shown in FIG. 4. The total 789 bp DNA sequence of the BstEII-Xmnl fragment of pAA130 is shown in FIG. 5. The ORF is underlined as shown in FIG. 6. Thus ORF encodes a polypeptide of 154 amino acids. The amino terminal 18 residue peptide resembles a membrane transport signal sequence determined for other proteins (Watson, 1984, supra) In addition, sequence data from the TnPhoA fusions in pAA130 demonstrated that all three transpositions were into the reading frame of the 154 amino acid polypeptide.

The amino acid composition of the proposed mature gene product as deduced from the DNA sequence of the ORF of pAA130 is shown in Table 1.

**TABLE 1**

| DEDUCED AMINO ACID COMPOSITIONS OF MATURE PBOMP-2 | |
|---|---|
| Amino Acid Residues | Mature PBOMP-2 of pAA130^{a} |
| Aspartic Acid | 6 |
| Asparagine | 4 |
| Threonine | 5 |
| Serine | 13 |
| Glutamic Acid | 5 |
| Glutamine | 7 |
| Proline | 1 |
| Glycine | 24 |
| Alanine | 16 |
| Cysteine | 1 |
| Valine | 18 |
| Methionine | 1 |
| Isoleucine | 13 |
| Leucine | 5 |
| Tyrosine | 2 |
| Phenylalanine | 2 |
| Lysine | 7 |
| Histidine | 0 |
| Arginine | 6 |
| Tryptophan | 0 |

| | |
|---|---|
| **a** The apparent molecular weight of mature PBOMP-2 was 13,461. The number of amino acid residues was 136. | |

In addition, although the product encoded by this gene is recognized by polyclonal anti-PBOMP-1 antisera, it is not recognized by monoclonal antibodies to PBOMP-1. From these observations, it is clear that the Hi gene expressed by pAA130 is not the gene for PBOMP-1. Thus the PBOMP-gene encoded by pAA130 was designated PBOMP-2.

### 6.5. CHARACTERIZATION OF PBOMPs EXPRESSED BY RECOMBINANT E. COLI AS LIPOPROTEINS

The following experiments were performed to investigate whether PBOMPs expressed by recombinant E. coli also exist as lipoproteins. Two different in vivo methods were used to verify the lipoprotein nature of the expressed PBOMPs.

In one series of experiments, cells containing recombinant plasmids expressing PBOMPs were cultured in the presence of a radioactively labelled fatty acid. Under such conditions, any lipoprotein formed containing the covalently attached fatty acid will be specifically radiolabelled.

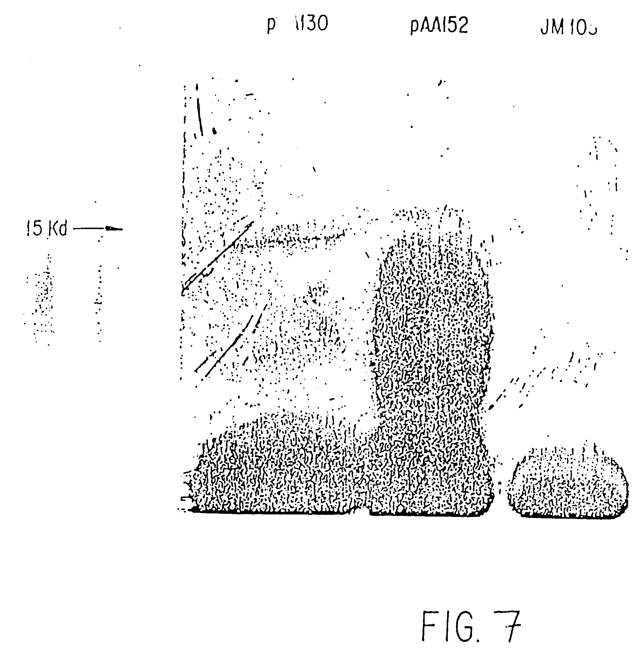

E. coli JM83 cells containing plasmid pAA130 expressing PBOMP-2 were grown for 2 hours in M9-minimal medium containing 50 uCi/ml ¹⁴C-palmitic acid. Whole cell lysates (10,000 trichloroacetic acid precipitable cpm/lane) were electrophoresed on 15% SDS-PAGE gels at 35 mA constant current. The gels were impregnated with sodium salicylate (5 x gel volume x 1 M solution for 20 minutes), dried, and exposed at -70°C on XAR-5 film (Eastman Kodak Company, Rochester, N. Y). Whole cell lysates of normal E. coli JM83 cells similarly cultured were run as controls. Results obtained are illustrated in FIG. 7.

As demonstrated in FIG. 7, a radiolabelled protein of about 15,000 daltons was observed in lysates of E. coli containing pAA130 which was not observed in lysates of control E. coli cells. Thus, the PBOMP-2 expressed by the recombinant E. coli were specifically radiolabelled.

In another series of experiments, cells containing recombinant plasmids expressing PBOMPs were cultured in the presence of globomycin, an antibiotic known to specifically block processing of all known bacterial lipoproteins by inhibition of the lipoprotein signal peptidase (Inukai et al., 1978, J. Antibiotics (Tokyo) 31:1203-1205).

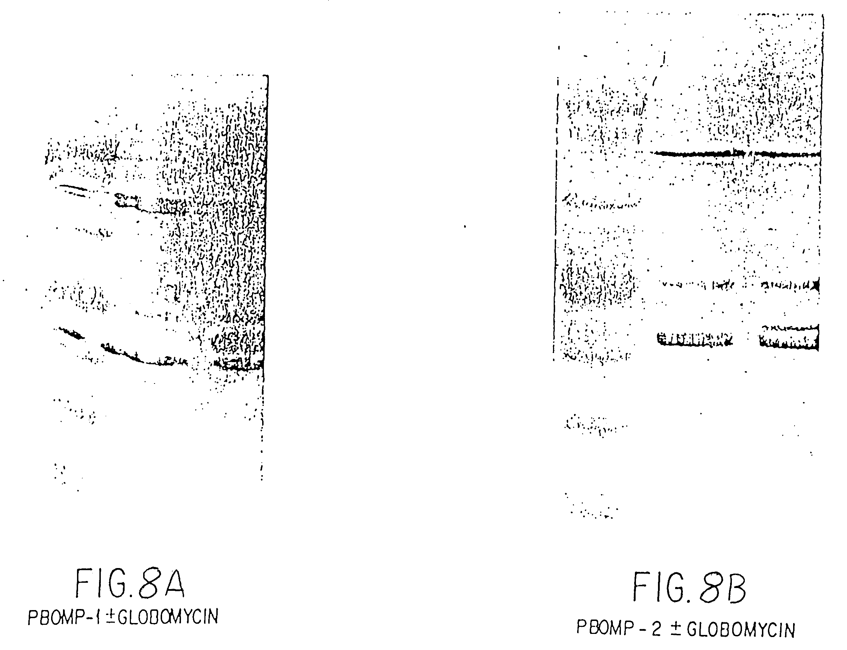

E. coli JM83 cells containing pAA130 were cultured in the presence of 25 ug/ml globomycin (obtained from Dr. M. Inouye, Robert Woods Johnson Medical Dental School, Piscataway, N.J.) for 1 hour. Similar cultures untreated with globomycin served as controls. Whole cell lysates were electrophoresed on 15% SDS-PAGE gels, transferred to nitrocellulose, and probed with polyclonal anti-PBOMP-1 antisera. Results obtained are illustrated in FIG. 8.

As demonstrated in FIG. 8, in lysates of globmycin-treated cells expressing PBOMP-2, an approximately 16,500 dalton band was observed which was not detected in lysates of control or untreated cells.

Based on results obtained in both series of in vivo experiments, the PBOMP-2 proteins expressed by recombinant E. coli containing PBOMP genes are lipoproteins.

### 7. NOVEL PLASMIDS FOR ENHANCED EXPRESSION OF PBOMPS IN E. COLI

### 7.1. ENHANCED EXPRESSION OF PBOMP-2 IN E. COLI

PBOMP-2 is also expressed at low levels (less than 1% of cell protein) from plasmid pAA130. Expression of PBOMP-2 is apparently under control of its native promoter.

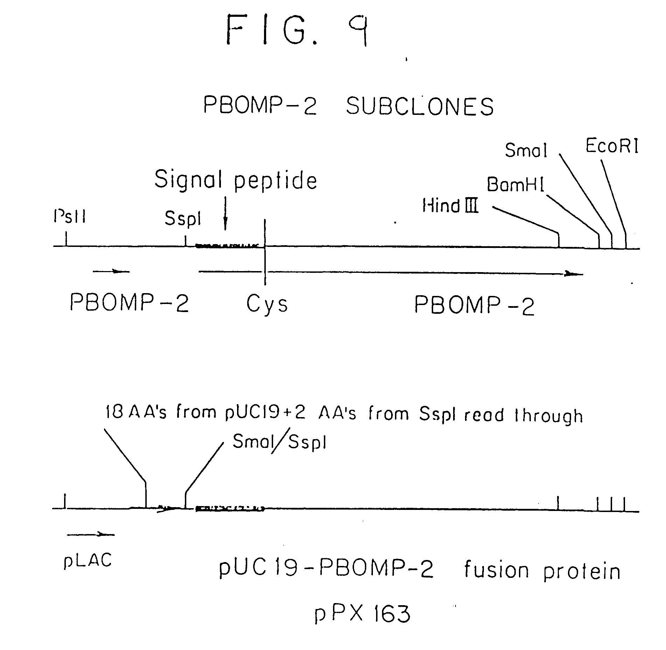

In order to improve the yield of PBOMP-2, the PBOMP-2 gene in pAA130 was cleaved at a unique SspI restriction site 5 bases 5' to the PBOMP-2 initiation codon and ligated to the SmaI site of pUC19 (FIG. 9). The litigation results in a hybrid open reading frame (ORF) containing the entire PBOMP-2 ORF (including the signal sequence) plus 18 codons from pUC19 and 2 codons from the gene fusion site. The protein of this fusion gene has a predicted molecular weight of 17,999 daltons; and is expressed under regulation of lac promoter. This plasmid is designated pPX163.

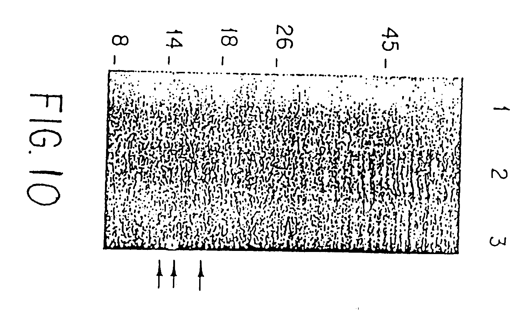

When plasmid pPX163 was transformed into E. coli JM103 and induced with IPTG, a protein of 17,500 daltons apparent molecular weight was expressed. In addition, a protein doublet at 15,000 daltons molecular weight was observed. All three proteins were recognized by anti-PBOMP-1 antisera on Western Blot (FIG. 10). By Coomassie blue staining of SDS-PAGE, the three forms of recombinant PBOMP-2 comprised approximately 15% of total cell protein after lac induction (FIG. 10, Lane 3). The three forms of recombinant PBOMP-2 expressed from pPX163 have been isolated and N-terminal peptide analysis reveals that:
1. The 17,500 dalton apparent MW band is the predicted pUC19/PBOMP-2 hybrid protein;
2. The larger 15,000 dalton MW band starts at the first methionine residue of the PBOMP-2 signal sequence (i.e. the initiation codon of the PBOMP-2 ORF); and is apparently due to reinitiation of translation at this point; and
3. The lower 15,000 dalton MW band is blocked to terminal analysis and can be labeled with ¹⁴C-palmitate. Hence, this band consists of lipoprotein processed PBOMP-2.

### 8. DEPOSIT OF MICROORGANISMS

The following E. coli strains carrying the listed plasmids have been deposited with the Agricultural Research Culture Collection (NRRL), Peoria, IL. and have been assigned the following accession numbers:

| E. Coli Strain | Plasmid | Accession Number |
|---|---|---|
| JM 83 | pAA130 | B-18154 |
| JM 83 | pGD103 | B-18153 |
| JM 103 | pPX163 | B-18285 |

The present invention is not to be limited in scope by the microorganisms deposited since the deposited embodiment is intended as a single illustration of one aspect of the invention and the many microorganisms which are functionally equivalent are within the scope of the present invention. Indeed, various modifications of the invention in addition to those shown and described herein will become apparent to those skilled in the art from the foregoing description and accompanying figures. Such modifications are intended to fall within the scope of the appended claims.

It is also to be understood that all base pair sizes given for nucleotides are approximate and are used for purposes of description.

## Claims

1. A substantially pure antigenic peptide or protein related to an epitope of Haemophilus influenzae which comprises an outer membrane protein, in which the outer membrane protein is an approximately 16000 dalton molecular weight outer membrane protein having an amino acid sequence comprising the amino acid sequence substantially as depicted in Fig. 6 or any antigenic portion thereof.

2. The peptide or protein according to claim 1, having an amino acid sequence comprising the amino acid sequence as depicted in Fig. 6 from amino acid residue 19 to 154 or any antigenic portion thereof.

3. The peptide or protein according of claim 1 or 2, in which the peptide or protein was purified from a cultured cell containing a nucleotide sequence encoding the peptide or protein which is under control of a second nucleotide sequence that regulates gene expression so that the peptide or protein is expressed by the cultured cell.

4. The peptide or protein of claim 3, in which the cultured cell is a microorganism.

5. The peptide or protein of claim 4, in which the microorganism is a bacterium.

6. The peptide or protein of claim 4, in which the microorganism is a yeast.

7. The peptide or protein of claim 3, in which the cultured cell is an animal cell line.

8. The peptide or protein of claim 3, in which the cultured cell is an insect cell line.

9. The peptide or protein according to claim 1 or 2, in which the peptide or protein was chemically synthesized.

10. The peptide or protein according to claim 1 or 2, in which the peptide or protein was obtained from a cell culture of Haemophilus influenzae by a process comprising the steps of :
(a) isolating an outer membrane protein enriched insoluble cell wall fraction from physically disrupted cells of Haemophilus influenzae ; and
(b) obtaining the peptide or protein in soluble form from the insoluble cell wall fraction by either : (i) heating the fraction in the presence of a detergent which is suitable for administration to a human, or (ii) digesting the fraction with lysosyme either in the presence or absence of said detergent.

11. A peptide or protein having one or more antigenic determinants of an Haemophilus influenzae outer membrane protein produced by an Escherichia coli bacterium deposited with the NRRL and assigned accession No. B-18154, recombinants and genetically engineered derivatives thereof.

12. A subunit vaccine formulation in which the immunogen comprises an effective amount of the peptide or protein of any one of claims 1 to 11, mixed with a pharmaceutical carrier.

13. A recombinant vector, comprising a DNA sequence coding for an antigenic terminant of an Haemophilus influenzae outer member protein, in which the outer membrane protein has an amino acid sequence substantially as depicted in Fig. 6 or any portion thereof comprising at least seven contiguous amino acids, said amino acid sequence containing one or more antigenic determinants of said Haemophilus influenzae outer membrane protein.

14. The recombinant vector according to claim 13, in which the DNA sequence is under control of expression control elements.

15. The recombinant vector according to claim 13 or 14, in which the vector is pAA130 as depicted in Figure 2 or pPX163 as depicted in figure 9, recombinants or genetically engineered derivatives thereof.

16. A unicellular organism containing the recombinant vector of any one of claims 13 to 15.

17. A bacterium containing the recombinant vector of any one of claims 13 to 16.

18. A bacterium according to claim 17, comprising an Escherichia coli bacterium deposited with the NRRL and assigned accession No. B-18154, No. B-18285, recombinants and genetically engineered derivatives thereof.

19. A method for the detection of Haemophilus influenzae in a sample of tissue or body fluids, comprising : using the peptide or protein according to any one of claims 1 to 11 as an antigen in an assay system selected from the group consisting of an enzyme-linked immunosorbent assay, a radioimmunoassay, a gel diffusion pricipitin reaction assay, an immunodiffusion assay, an agglutination assay, a fluorescent immunoassay, a protein A immunoassay and an immunoelectrophoresis assay ; and detecting any reaction with the antigen, in which any reaction indicated the presence of Haemophilus influenzae in the sample.

20. A method for the detection of Haemophilus influenzae in a sample of tissue or body fluids, comprising : using a nucleotide sequence of a gene encoding a peptide or protein related to an outer membrane protein of Haemophilus influenzae as a depicted in Fig. 6 or a fragment thereof as a probe in a nucleic acid hybridization assay and detecting any reaction with the probe, in which any reaction indicates the presence of Haemophilus influenzae in the sample.

21. The method according to claim 20, in which the nucleic acid hybridization assay is selected from the group consisting of Southern blots, Northern blots and colony blots.

## Patentansprüche

1. Ein im wesentlichen reines antigenes Peptid oder Protein mit Verbindung zu einem Epitop von Haemophilus influenzae, das ein äußeres Membranprotein, in dem das äußere Membranprotein ein äußeres Membranprotein mit ungefährem Molekulargewicht von 16 000 Dalton mit einer Aminosäuresequenz, die im wesentlichen die Aminosäuresequenz wie in Fig. 15 dargestellt aufweist oder irgendeinen antigenen Abschnitt hiervon umfaßt.

2. Peptid oder Protein nach Anspruch 1 mit einer Aminosäuresequenz, die die Aminosäuresequenz wie in Fig. 15 dargestellt von der Aminosäuregruppe 19 bis 154, oder irgendeinen antigenen Abschnitt hiervon umfaßt.

3. Peptid oder Protein nach Anspruch 1 oder 2, in dem das Peptid oder Protein aus einer Zellkultur aufgereinigt wurde, die eine das Peptid oder Protein codierende Nucleotidsequenz enthält, die unter Kontrolle einer zweiten Nucleotidsequenz steht, das die Genexpression reguliert, so daß das Peptid oder Protein durch die Zellkultur aufgebaut wird.

4. Peptid oder Protein nach Anspruch 3, in dem die Zellkultur ein Mikroorganismus ist.

5. Peptid oder Protein nach Anspruch 4, in dem der Mikroorganismus ein Bakterium ist.

6. Peptid oder Protein nach Anspruch 4, in dem der Mikroorganismus ein Hefepilz ist.

7. Peptid oder Protein nach Anspruch 3, in dem die Zellkultur eine tierische Zellinie ist.

8. Peptid oder Protein nach Anspruch 3, in dem die Zellkultur eine Insekten-Zellinie ist.

9. Peptid oder Protein nach Anspruch 1 oder 2, in dem das Peptid oder Protein chemisch synthetisiert wurde.

10. Peptid oder Protein nach Anspruch 1 oder 2, in dem das Peptid oder Protein aus einer Zellkultur von Haemophilius influenzae durch ein Verfahren erhalten wurde, das die Schritte aufweist:
(a) Isolierung von mit äußerem Membranprotein angereichertem unlöslichem Zellwandanteil physikalisch zerstörter Zellen von Haemophilius influenzae: und
(b) Erhalten des Peptids oder Proteins in löslicher Form aus dem unlöslichen Zellwandanteil entweder durch: (i) Erhitzen des Anteils in Gegenwart eines Detergens, das für die Verabreichung an einen Menschen geeignet ist, oder (ii) Digerieren des Anteils mit Lysozym entweder in Gegenwart oder Abwesenheit des Detergens.

11. Peptid oder Protein mit einem oder mehreren antigenen Determinanten eines äußeren Membranproteins von Haemophilius influenzae, hergestellt durch ein Escherichia coli Bakterium, hinterlegt bei der NRRL mit der zugeordneten Zugangs-Nr. B-18154, sowie rekombinante und gentechnische Derivate hiervon.

12. Formulierung einer Vakzine-Untereinheit, in der das Immungen eine wirksame Menge des Peptids oder Proteins nach irgendeinem der Ansprüche 1 bis 11 gemischt mit einem pharmazeutischen Träger umfaßt.

13. Rekombinanter Vektor, der eine DNA-Sequenz-Codierung für ein antigenes Terminant eines äußeren Membranproteins von Haemophilius influenzae umfaßt, in dem das äußere Membranprotein eine Aminosäuresequenz im wesentlichen wie in Fig. 15 dargestellt besitzt oder irgendein Abschnitt hiervon, der mindestens sieben benachbarte Aminosäuren umfaßt, wobei die Aminosäuresequenz eine oder mehr antigene Determinanten des äußeren Membranproteins von Haemophilius influenzae enthält.

14. Rekombinanter Vektor nach Anspruch 13, in dem die DNA-Sequenz sich unter Kontrolle von Expressionssteuerelementen befindet.

15. Rekombinanter Vektor nach Anspruch 13 oder 14, in dem der Vektor pAA130 wie in Figur 2 oder pPX163 wie in Figur 9 dargestellt ist, sowie rekombinante oder gentechnische Derivate hiervon.

16. Einzelliger Organismus, der den rekombinanten Vektor nach irgendeinem der Ansprüche 13 bis 15 enthält.

17. Bakterium, das den rekombinanten Vektor nach irgendeinem der Ansprüche 13 bis 16 enthält.

18. Bakterium nach Anspruch 17, das ein Escherichia coli Bakterium, hinterlegt bei der NRRL mit den zugeordneten Zugangs-Nr. B-18154, Nr. B-18285, sowie rekombinante und gentechnische Derivate hiervon umfaßt.

19. Verfahren zur Detektion von Haemophilius influenzae in einer Gewebs-oder Körperflüssigkeitsprobe, das aufweist: Verwendung des Peptids oder Proteins nach irgendeinem der Ansprüche 1 bis 11 als ein Antigen in einem Assay-System, ausgewählt aus der Gruppe, bestehend aus einem Enzymimmuntest (enzyme-linked immunosorbent assay), einem Radioimmunoassay, einem Geldiffusionspräzipitin-Reaktionsassay, einem Immunodiffusionsassay, einem Agglutinationsassay, einem Fluoreszenz-Immunoassay, einem Protein A Immunoassay und einem Immunoelektrophoreseassay; und Detektion jeder Reaktion mit dem Antigen, wobei jede Reaktion die Gegenwart von Haemophilius influenzae in der Probe anzeigt.

20. Verfahren zur Detektion von Haemophilius influenzae in einer Gewebs-oder Körperflüssigkeitsprobe, das aufweist: Verwendung einer ein Peptid oder Protein codierenden Nucleotidsequenz eines Gens, das mit Verbindung zu einem äußeren Membranprotein von Haemophilius influenzae wie in Fig. 15 dargestellt oder einem Fragment hiervon als Sonde in einem Nucleinsäurehybridisierungstest und Detektion jeder Reaktion mit der Sonde, in der jede Reaktion die Gegenwart von Haemophilius influenzae in der Probe anzeigt.

21. Verfahren nach Anspruch 20, in dem der Nucleinsäurehybridisierungstest ausgewählt ist aus der Gruppe, bestehend aus Southern-Blots, Northern-Blots und Kolonie-Blots.

## Revendications

1. Peptide ou protéine antigène, essentiellement pur, apparenté à un épitope de Haemophilus influenzae, qui comprend une protéine de membrane externe, dans lequel la protéine de membrane externe est une protéine de membrane externe de masse moléculaire voisine de 16 000 daltons ayant une séquence d'acides aminés comprenant la séquence d'acides aminés essentiellement telle que décrite sur la figure 6 ou une partie antigène quelconque de celle-ci.

2. Peptide ou protéine selon la revendication 1, ayant une séquence d'acides aminés comprenant la séquence d'acides aminés essentiellement telle que décrite sur la figure 6 depuis le résidu d'acide aminé 19 à 154 ou une partie antigène quelconque de celle-ci.

3. Peptide ou protéine selon la revendication 1 ou 2, dans lequel le peptide ou la protéine a été purifié à partir d'une cellule cultivée contenant une séquence nucléotidique codant pour le peptide ou la protéine qui est sous le contrôle d'une seconde séquence nucléotidique qui régule l'expression du gène de sorte que le peptide ou la protéine est exprimé par la cellule cultivée.

4. Peptide ou protéine selon la revendication 3, dans lequel la cellule cultivée est un microorganisme.

5. Peptide ou protéine selon la revendication 4, dans lequel le microorganisme est une bactérie.

6. Peptide ou protéine selon la revendication 4, dans lequel le microorganisme est une levure.

7. Peptide ou protéine selon la revendication 3, dans lequel la cellule cultivée est une lignée cellulaire animale.

8. Peptide ou protéine selon la revendication 3, dans lequel la cellule cultivée est une lignée cellulaire d'insecte.

9. Peptide ou protéine selon la revendication 1 ou 2, dans lequel le peptide ou la protéine est synthétisé par voie chimique.

10. Peptide ou protéine selon la revendication 1 ou 2, dans lequel le peptide ou la protéine est obtenu à partir d'une culture cellulaire de Haemophilus influenzae par un procédé comprenant les étapes consistant à:
(a) isoler une fraction de paroi cellulaire insoluble enrichie en protéine de membrane externe tirée de cellules de Haemophilus influenzae rompues physiquement; et
(b) obtenir le peptide ou la protéine sous forme soluble à partir de la fraction de paroi cellulaire insoluble, soit par (i) chauffage de la fraction en présence d'un détergent convenable pour l'administration à un être humain, soit par (ii) digestion de la fraction avec un lysozyme, en présence ou en l'absence dudit détergent.

11. Peptide ou protéine possédant un ou plusieurs déterminants antigènes d'une protéine de membrane externe de Haemophilus influenzae produite par une bactérie Escherichia coli déposée auprès de la NRRL, sous le n° d'accès B-18154, ses recombinants et ses dérivés manipulés génétiquement.

12. Formulation de vaccin sous-unitaire dans laquelle l'immunogène comprend une quantité efficace du peptide ou de la protéine selon l'une quelconque des revendications 1 à 11, mélangée avec un véhicule pharmaceutique.

13. Vecteur recombinant, comprenant une séquence d'ADN codant pour un déterminant antigène d'une protéine de membrane externe de Haemophilus influenzae, dans lequel la protéine de membrane externe possède une séquence d'acides aminés essentiellement telle que décrite sur la figure 6 ou toute portion de celle-ci comprenant au moins sept acides aminés contigus, ladite séquence d'acides aminés contenant un ou plusieurs déterminants antigènes de ladite protéine de membrane externe de Haemophilus influenzae.

14. Vecteur recombinant selon la revendication 13, dans lequel la séquence d'ADN est sous le contrôle d'éléments de contrôle de l'expression.

15. Vecteur recombinant selon la revendication 13 ou 14, dans lequel le vecteur est pAA130 tel que représenté sur la figure 2 ou pPX163 tel que représenté sur la figure 9, leurs recombinants ou leurs dérivés manipulés génétiquement.

16. Organisme unicellulaire contenant le vecteur recombinant selon l'une quelconque des revendications 13 à 15.

17. Bactérie contenant le vecteur recombinant selon l'une quelconque des revendications 13 à 16.

18. Bactérie selon la revendication 17, comprenant une bactérie Escherichia coli déposée auprès de la NRRL, sous les n° d'accès B-18154, B-18285, ses recombinants et ses dérivés manipulés génétiquement.

19. Procédé de détection de Haemophilus influenzae dans un échantillon de tissu ou de fluides corporels, comprenant: l'utilisation du peptide ou de la protéine selon l'une quelconque des revendications 1 à 11 comme antigène dans un système d'essai choisi dans le groupe constitué par un essai par immunosorbant lié à une enzyme, un essai radioimmunologique, un essai de réaction à la précipitine par diffusion sur gel, un essai d'immunodiffusion, un essai d'agglutination, un immuno-essai fluorescent, un immunoessai à la protéine A et un essai d'immunoélectrophorèse; et la détection de la réaction éventuelle avec l'antigène, dans lequel la réaction indique la présence de Haemophilus influenzae dans l'échantillon.

20. Procédé de détection de Haemophilus influenzae dans un échantillon de tissu ou de fluides corporels, comprenant: l'utilisation d'une séquence nucléotidique d'un gène codant pour un peptide ou une protéine apparenté à une protéine de membrane externe de Haemophilus influenzae telle que décrite sur la figure 6 ou un fragment de celle-ci comme sonde dans un essai d'hybridation des acides nucléiques et la détection de la réaction éventuelle avec la sonde, dans lequel la réaction indique la présence de Haemophilus influenzae dans l'échantillon.

21. Procédé selon la revendication 20, dans lequel l'essai d'hybridation des acides nucléiques est choisi dans le groupe constitué par des Southern blots, des Northern blots et des analyses par transfert de colonies.
